(19) Europäisches Patentamt
European Patent Office
Office européen des brevets

(11) EP 4 406 974 A1

(12) **EUROPEAN PATENT APPLICATION**
published in accordance with Art. 153(4) EPC

(43) Date of publication:
**31.07.2024 Bulletin 2024/31**

(21) Application number: **22872048.8**

(22) Date of filing: **22.09.2022**

(51) International Patent Classification (IPC):
***C07K 16/30*** *(2006.01)* ***A61K 47/68*** *(2017.01)*
***A61K 31/4745*** *(2006.01)* ***A61P 35/00*** *(2006.01)*

(52) Cooperative Patent Classification (CPC):
**A61K 31/4745; A61K 47/68; A61P 35/00; C07K 16/30**

(86) International application number:
**PCT/CN2022/120481**

(87) International publication number:
**WO 2023/046003 (30.03.2023 Gazette 2023/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.09.2021 CN 202111116842**

(71) Applicants:
- **Shanghai Hansoh Biomedical Co., Ltd.**
  **Shanghai 201203 (CN)**
- **Jiangsu Hansoh Pharmaceutical Group Co., Ltd.**
  **Lianyungang, Jiangsu 222047 (CN)**
- **Changzhou Hansoh Pharmaceutical Co., Ltd.**
  **Xinbei District**
  **Changzhou**
  **Jiangsu 213001 (CN)**

(72) Inventors:
- **MAO, Dongjie**
  **Shanghai 201203 (CN)**
- **XIE, Yuejun**
  **Shanghai 201203 (CN)**
- **CHEN, Lili**
  **Shanghai 201203 (CN)**

(74) Representative: **EP&C**
**P.O. Box 3241**
**2280 GE Rijswijk (NL)**

(54) **ANTIBODY-DRUG CONJUGATE, PREPARATION METHOD THEREFOR, AND PHARMACEUTICAL USE THEREOF**

(57) Provided are an antibody-drug conjugate, a preparation method therefor, and a pharmaceutical use thereof, and specifically related are an anti-TROP-2 antibody-drug conjugate and a pharmaceutical use thereof. The antibody-drug conjugate is formed by connecting an anti-TROP-2 antibody or an antigen binding fragment thereof and an exitecan derivative by means of a linker, and the antibody-drug conjugate or a pharmaceutically acceptable salt or solvent compound thereof has a significant anti-tumor effect and good safety.

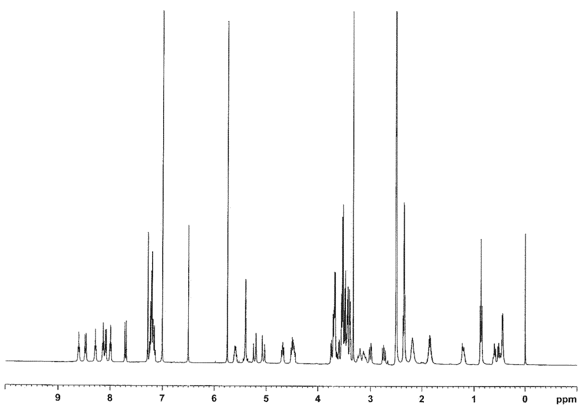


EP 4 406 974 A1

## Description

### FIELD OF THE INVENTION

The present invention belongs to the field of biomedicine. Specifically, the present invention relates to an anti-TROP-2 antibody conjugate and medical use thereof.

### BACKGROUND OF THE INVENTION

With the continuous deepening of research on tumor genomics, proteomics and signaling pathways, people have become increasingly clear about the interaction between oncogenes and tumor suppressor genes in tumor cells and their impact on the tumor microenvironment. This also makes it possible to design new anti-tumor treatment schemes for specific molecular targets of tumors.

Molecular targeted therapy of tumors is a new treatment model that is different from conventional surgery, radiotherapy, and chemotherapy and has advantages that a drug usually only bind to the corresponding target site, and kills or inhibits the target cells by directly affecting the function of a target molecule or via a physical or chemical effector molecule carried by it. Due to their clear target sites, this type of drugs usually have high selectivity and can effectively kill or inhibit target cells while producing no or only minor toxic side effects on normal tissue cells. Therefore, the development of molecular targeted drugs has become a hot topic in clinical cancer research.

Human trophoblast cell surface antigen 2 (TROP-2) is a cell surface glycoprotein encoded by the TACSTD2 gene. TROP-2 consists of 323 amino acids, including the signal peptide consists of 26 amino acids, the extracellular region consists of 248 amino acids, the transmembrane region consists of 23 amino acids, and the cytoplasmic region consists of 26 amino acids. There are 4 heterogeneous N-binding glycosylation sites in the extracellular domain of TROP-2. After adding carbohydrate chains, the apparent molecular weight increases by 11 to 13 KD. In the TACSTD gene family, the extracellular domain has a characteristic thyroglobulin (TY) sequence, which is generally considered to be related to the proliferation, infiltration, and metastasis of cancer cells.

A large number of clinical studies and literature reports have shown that TROP-2 is overexpressed in various carcinomas such as gastric cancer, lung cancer, large intestine cancer, ovarian cancer, breast cancer, prostate cancer, pancreatic cancer, liver cancer, and esophageal cancer. In contrast, TROP-2 is rarely or not expressed in normal tissues in adults, with a small amount of expression limited to cells in the epithelial area, and the expression level is also lower than that in cancer, indicating that TROP-2 is related to neoplasia. The overexpression of TROP-2 in tumor tissues is closely related to poor prognosis of patients and the metastasis of cancer cells, and also affects the overall survival rate of patients. Therefore, TROP-2 has become an attractive target in molecular targeted therapy of tumors.

Several studies on the antitumor effects of anti-hTROP-2 antibody have been reported:

US Patent No. 5840854 reports the cytotoxicity of a cytotoxin-conjugated anti-hTROP-2 monoclonal antibody (BR110) against human cancer cell lines H3619, H2987, MCF-7, H3396 and H2981.

U.S. Patent No. 6653104 discloses an antibody (RS7), which was tested in an *in vivo* model using an antibody labeled with radioactive substances. The antibody showed anti-tumor activity in a nude mouse xenograft model, but the anti-tumor effect of nude antibody alone was not reported.

U.S. Patent No. 7420040 also reports that an isolated monoclonal antibody produced by hybridoma cell line AR47A6.4.2 or AR52A301.5 obtained from immunizing mice with human ovarian cancer tissue was conjugated to hTROP-2, and showed anti-tumor activity in nude mice xenograft model.

CN 102827282A discloses a human anti-TROP-2 genetically engineered antibody IgG and use thereof. *In vitro* test results showed that the anti-TROP-2 antibody IgG had a significant inhibitory effect on the proliferation of pancreatic cancer cells.

CN 104114580A discloses an antibody (especially a humanized antibody) that specifically reacts with hTROP-2 and has anti-tumor activity *in vivo,* as well as a hybridoma that produces the antibody, a complex of the antibody with a drug, a pharmaceutical composition for diagnosing or treating a tumor, a method for detecting a tumor, and a kit for detecting or diagnosing a tumor.

However, it is very difficult to find monoclonal antibodies with high affinity, high specificity, and potent cytotoxicity or tumor killing/inhibiting/regressing activities. Therefore, there is still an urgent need to develop Trop-2 antibodies and other immunotherapeutic agents with good efficacy, high safety, and suitable for human patients.

### SUMMARY OF THE INVENTION

The object of the present invention is to provide an antibody-drug conjugate of general formula (I) or a pharmaceutically acceptable salt or solvent compound thereof,

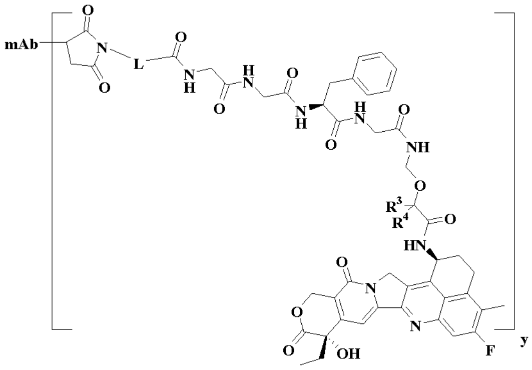

(I)

wherein:

L is $-(CR^1R^2)_m-[X_1-(CR^1R^2)_n-X_2]_t(CR^1R^2)_r-$;
$R^1$ or $R^2$ is each independently selected from the group consisting of hydrogen, deuterium, hydroxyl, amino, alkyl, halogen, haloalkyl, deuterated alkyl and hydroxyalkyl, preferably, $R^1$ or $R^2$ is hydrogen;
$X_1$ or $X_2$ is each independently selected from the group consisting of a bond, N, O and S; preferably, $X_1$ or $X_2$ is a bond or O,
m, n, r or t is each independently selected from the group consisting of 1, 2, 3 and 4; preferably, m, n, r or t is each independently selected from the group consisting of 1 and 2; $R^3$ or $R^4$ is each independently selected from the group consisting of hydrogen, halogen, haloalkyl, deuterated alkyl, cycloalkyl, heterocyclyl, aryl and heteroaryl;
or, $R^3$ and $R^4$ together with the carbon atom to which they are attached form cycloalkyl or heterocyclyl;
y is 1 to 20, preferably 1 to 10, more preferably 2 to 8, and further preferably 4, 6 or 8; mAb is an anti-TROP-2 antibody or an antigen binding fragment thereof.

In a preferred embodiment of the present invention, $R^1$ or $R^2$ is each independently selected from the group consisting of hydrogen, deuterium, hydroxyl, amino, $C_{1-3}$ alkyl, halogen, $C_{1-3}$ haloalkyl, $C_{1-3}$ deuterated alkyl and $C_{1-3}$ hydroxyalkyl, preferably, $R^1$ or $R^2$ is hydrogen; $X_1$ or $X_2$ is each independently selected from the group consisting of a bond, N, O and S; preferably, $X_1$ or $X_2$ is a bond or O,

m, n or r is each independently selected from the group consisting of 1, 2, 3 and 4; preferably, m, n or r is each independently selected from the group consisting of 1 and 2;
$R^3$ or $R^4$ is each independently selected from the group consisting of hydrogen, halogen, $C_{1-3}$ haloalkyl, $C_{1-3}$ deuterated alkyl, $C_{3-6}$ cycloalkyl, 4- to 8- membered heterocyclyl, $C_{5-10}$ aryl or 4- to 8- membered heteroaryl, preferably $R^3$ or $R^4$ is each independently selected from the group consisting of hydrogen and $C_{3-6}$ cycloalkyl;
or, $R^3$ and $R^4$ together with the carbon atom to which they are attached form $C_{3-6}$ cycloalkyl or 4- to 8- membered heterocyclyl.

In a further preferred embodiment of the present invention, the anti-TROP-2 antibody or the antigen binding fragment thereof comprises: HCDR1 as shown in SEQ ID NO: 3, HCDR2 as shown in RIDPXDSETHYNQKFKD and HCDR3 as shown in SEQ ID NO: 5; and LCDR1 as shown in SEQ ID NO: 6, LCDR2 as shown in SEQ ID NO: 7 and LCDR3 as shown in SEQ ID NO: 8,
**[0011]** X is selected from the group consisting of R, Y, Q, L, T, I, F, E and A.
In a preferred embodiment of the present invention, the TROP-2 antibody or the antigen binding fragment thereof comprises:

HCDR1 as shown in SEQ ID NO: 3, HCDR2 as shown in SEQ ID NO: 9 and HCDR3 as shown in SEQ ID NO: 5; and LCDR1 as shown in SEQ ID NO: 6, LCDR2 as shown in SEQ ID NO: 7 and LCDR3 as shown in SEQ ID NO: 8; or,
HCDR1 as shown in SEQ ID NO: 3, HCDR2 as shown in SEQ ID NO: 10 and HCDR3 as shown in SEQ ID NO: 5; and LCDR1 as shown in SEQ ID NO: 6, LCDR2 as shown in SEQ ID NO: 7 and LCDR3 as shown in SEQ ID NO: 8; or,
HCDR1 as shown in SEQ ID NO: 3, HCDR2 as shown in SEQ ID NO: 11 and HCDR3 as shown in SEQ ID NO: 5; and LCDR1 as shown in SEQ ID NO: 6, LCDR2 as shown in SEQ ID NO: 7 and LCDR3 as shown in SEQ ID NO: 8; or,

HCDR1 as shown in SEQ ID NO: 3, HCDR2 as shown in SEQ ID NO: 12 and HCDR3 as shown in SEQ ID NO: 5; and LCDR1 as shown in SEQ ID NO: 6, LCDR2 as shown in SEQ ID NO: 7 and LCDR3 as shown in SEQ ID NO: 8; or,
HCDR1 as shown in SEQ ID NO: 3, HCDR2 as shown in SEQ ID NO: 13 and HCDR3 as shown in SEQ ID NO: 5; and LCDR1 as shown in SEQ ID NO: 6, LCDR2 as shown in SEQ ID NO: 7 and LCDR3 as shown in SEQ ID NO: 8; or,
HCDR1 as shown in SEQ ID NO: 3, HCDR2 as shown in SEQ ID NO: 14 and HCDR3 as shown in SEQ ID NO: 5; and LCDR1 as shown in SEQ ID NO: 6, LCDR2 as shown in SEQ ID NO: 7 and LCDR3 as shown in SEQ ID NO: 8; or,
HCDR1 as shown in SEQ ID NO: 3, HCDR2 as shown in SEQ ID NO: 15 and HCDR3 as shown in SEQ ID NO: 5; and LCDR1 as shown in SEQ ID NO: 6, LCDR2 as shown in SEQ ID NO: 7 and LCDR3 as shown in SEQ ID NO: 8; or,
HCDR1 as shown in SEQ ID NO: 3, HCDR2 as shown in SEQ ID NO: 16 and HCDR3 as shown in SEQ ID NO: 5; and LCDR1 as shown in SEQ ID NO: 6, LCDR2 as shown in SEQ ID NO: 7 and LCDR3 as shown in SEQ ID NO: 8; or,
HCDR1 as shown in SEQ ID NO: 3, HCDR2 as shown in SEQ ID NO: 17 and HCDR3 as shown in SEQ ID NO: 5; and LCDR1 as shown in SEQ ID NO: 6, LCDR2 as shown in SEQ ID NO: 7 and LCDR3 as shown in SEQ ID NO: 8.

In a preferred embodiment of the present invention for the antibody-drug conjugate or the pharmaceutically acceptable salt or solvent compound thereof according to the present invention, the anti-TROP-2 antibody or the antigen binding fragment thereof is selected from the group consisting of a murine antibody or an antigen binding fragment thereof, a chimeric antibody or an antigen binding fragment thereof, a human antibody or an antigen binding fragment thereof, a humanized antibody or an antigen binding fragment thereof.

In a preferred embodiment of the present invention for the antibody-drug conjugate or the pharmaceutically acceptable salt or solvent compound thereof according to the present invention, the anti-TROP-2 antibody or the antigen binding fragment thereof further comprises a heavy chain constant region derived from human IgG1, IgG2, IgG3 or IgG4, or a variant thereof.

In a further preferred embodiment of the present invention, the anti-TROP-2 antibody or the antigen binding fragment thereof further comprises a heavy chain constant region derived from human IgG1, IgG2 or IgG4, or a variant thereof.

In a further preferred embodiment of the present invention, the anti-TROP-2 antibody or the antigen binding fragment thereof further comprises a heavy chain constant region as shown in SEQ ID NO: 1.

In a preferred embodiment of the present invention for the antibody-drug conjugate or the pharmaceutically acceptable salt or solvent compound thereof according to the present invention, the anti-TROP-2 antibody or the antigen binding fragment thereof further comprises a light chain constant region derived from a human antibody kappa chain or lambda chain, or a variant thereof.

In a further preferred embodiment of the present invention, the anti-TROP-2 antibody or the antigen binding fragment thereof further comprises a light chain constant region derived from a human antibody kappa chain.

In a further preferred embodiment of the present invention, the anti-TROP-2 antibody or the antigen binding fragment thereof further comprises a light chain constant region as shown in SEQ ID NO: 2.

In a preferred embodiment of the present invention, the anti-TROP-2 antibody or the antigen binding fragment thereof comprises a heavy chain variable region selected from the group consisting of the following sequence, or a heavy chain variable region having at least 70%, 75%, 80%, 85%, 90%, 95% or 99% identity to the following sequence: SEQ ID NO: 18, SEQ ID NO: 20, SEQ ID NO: 21, SEQ ID NO: 22, SEQ ID NO: 23, SEQ ID NO: 24, SEQ ID NO: 25, SEQ ID NO: 26 or SEQ ID NO: 27.

In a preferred embodiment of the present invention for the antibody-drug conjugate or the pharmaceutically acceptable salt or solvent compound thereof according to the present invention, wherein the anti-TROP-2 antibody or the antigen binding fragment thereof comprises a the light chain variable region of or having at least 70%, 75%, 80%, 85%, 90%, 95% or 99% identity to SEQ ID NO: 19.

In a further preferred embodiment of the present invention, the anti-TROP-2 antibody or the antigen binding fragment thereof comprises:

a heavy chain variable region as shown in SEQ ID NO: 18 and a light chain variable region as shown in SEQ ID NO: 19; or,
a heavy chain variable region as shown in SEQ ID NO: 20 and a light chain variable region as shown in SEQ ID NO: 19; or,
a heavy chain variable region as shown in SEQ ID NO: 21 and a light chain variable region as shown in SEQ ID NO: 19; or,
a heavy chain variable region as shown in SEQ ID NO: 22 and a light chain variable region as shown in SEQ ID NO: 19; or,
a heavy chain variable region as shown in SEQ ID NO: 23 and a light chain variable region as shown in SEQ ID NO: 19; or,
a heavy chain variable region as shown in SEQ ID NO: 24 and a light chain variable region as shown in SEQ ID

NO: 19; or,
a heavy chain variable region as shown in SEQ ID NO: 25 and a light chain variable region as shown in SEQ ID NO: 19; or,
a heavy chain variable region as shown in SEQ ID NO: 26 and a light chain variable region as shown in SEQ ID NO: 19; or,
a heavy chain variable region as shown in SEQ ID NO: 27 and a light chain variable region as shown in SEQ ID NO: 19.

In a preferred embodiment of the present invention, the anti-TROP-2 antibody or the antigen binding fragment thereof comprises a heavy chain selected from the group consisting of the following sequence, or a heavy chain having at least 80%, 85%, 90%, 95% or 99% identity to the following sequence: SEQ ID NO: 28, SEQ ID NO: 30, SEQ ID NO: 31, SEQ ID NO: 32, SEQ ID NO: 33, SEQ ID NO: 34, SEQ ID NO: 35, SEQ ID NO: 36 or SEQ ID NO: 37.

In a preferred embodiment of the present invention, the anti-TROP-2 antibody or the antigen binding fragment thereof comprises a light chain selected from the group consisting of the following sequence, or a light chain having at least 80%, 85%, 90%, 95% or 99% identity to the following sequence: SEQ ID NO: 29.

In a preferred embodiment of the present invention, the anti-TROP-2 antibody or the antigen binding fragment thereof comprises:

a heavy chain as shown in SEQ ID NO: 28 and a light chain as shown in SEQ ID NO: 29; or,
a heavy chain as shown in SEQ ID NO: 30 and a light chain as shown in SEQ ID NO: 29; or,
a heavy chain as shown in SEQ ID NO: 31 and a light chain as shown in SEQ ID NO: 29; or,
a heavy chain as shown in SEQ ID NO: 32 and a light chain as shown in SEQ ID NO: 29; or,
a heavy chain as shown in SEQ ID NO: 33 and a light chain as shown in SEQ ID NO: 29; or,
a heavy chain as shown in SEQ ID NO: 34 and a light chain as shown in SEQ ID NO: 29; or,
a heavy chain as shown in SEQ ID NO: 35 and a light chain as shown in SEQ ID NO: 29; or,
a heavy chain as shown in SEQ ID NO: 36 and a light chain as shown in SEQ ID NO: 29; or,
a heavy chain as shown in SEQ ID NO: 37 and a light chain as shown in SEQ ID NO: 29.

In a further preferred embodiment of the present invention for the antibody-drug conjugate or the pharmaceutically acceptable salt or solvent compound thereof according to the present invention, the antibody-drug conjugate or the pharmaceutically acceptable salt or solvent compound thereof is selected from the group consisting of an antibody-drug conjugate of general formula (II) or a pharmaceutically acceptable salt, solvent compound, tautomer, mesomer, racemate, enantiomer, or diastereomer thereof, and a mixture thereof:

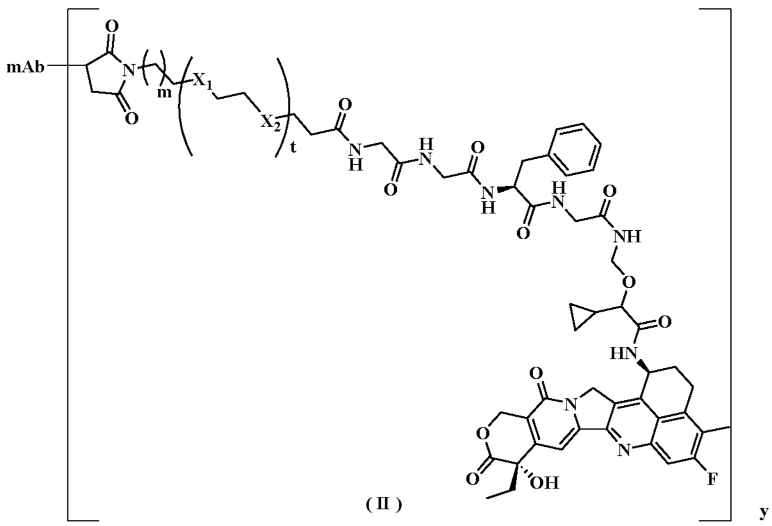

(II)

$X_1$ or $X_2$ is a bond or O;
m is 0 or 1; and
t is 1 or 2.

In a further preferred embodiment of the present invention for the antibody-drug conjugate or the pharmaceutically acceptable salt or solvent compound thereof according to the present invention, the antibody-drug conjugate or the pharmaceutically acceptable salt or solvent compound thereof is selected from the group consisting of an antibody-drug conjugate of general formula (III) or a pharmaceutically acceptable salt or solvent compound thereof:

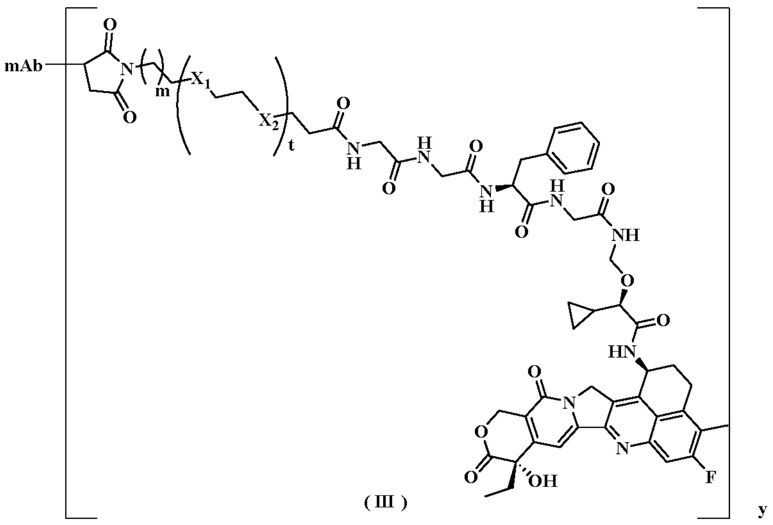

(III)

mAb is selected from the group consisting of the above-mentioned anti-TROP-2 antibody or antigen binding fragment thereof,

y is selected from the group consisting of 2-10, preferably 4-10, more preferably 4, 6, 8 or 10.

In a further preferred embodiment of the present invention, the antibody-drug conjugate or the pharmaceutically acceptable salt or solvent compound thereof is selected from the group consisting of the following structures:

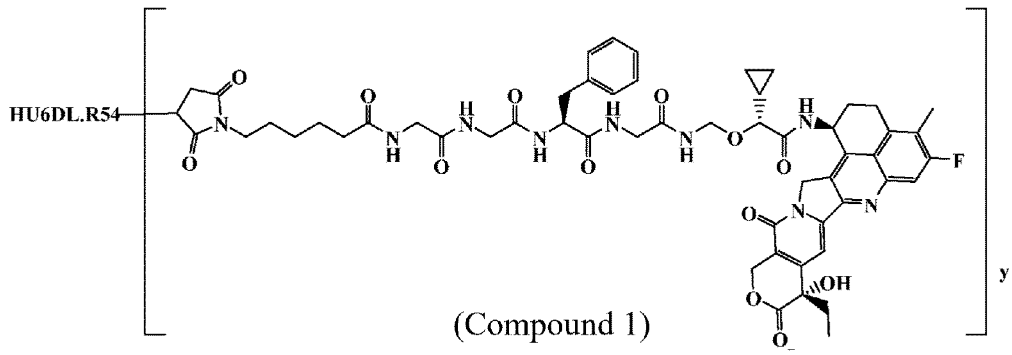

(Compound 1)

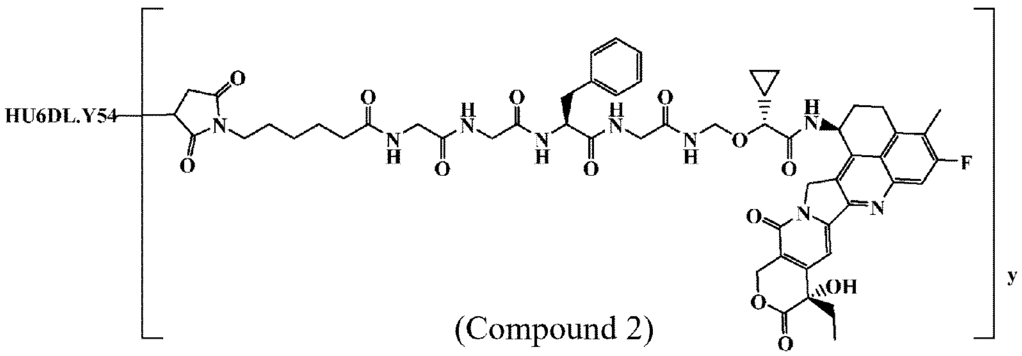

(Compound 2)

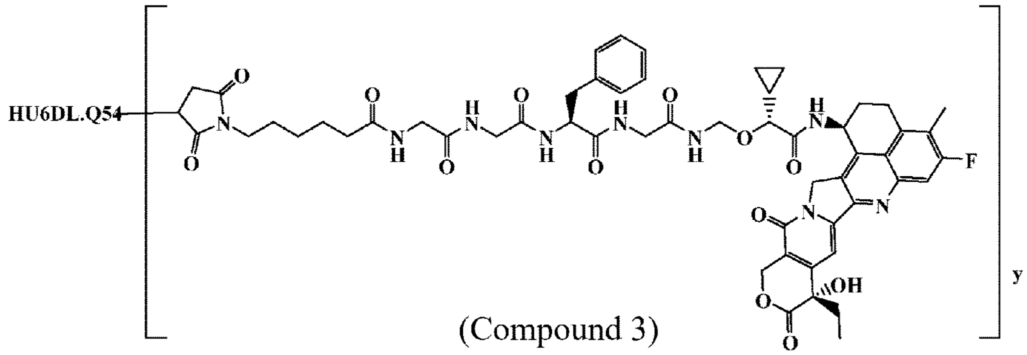

(Compound 3)

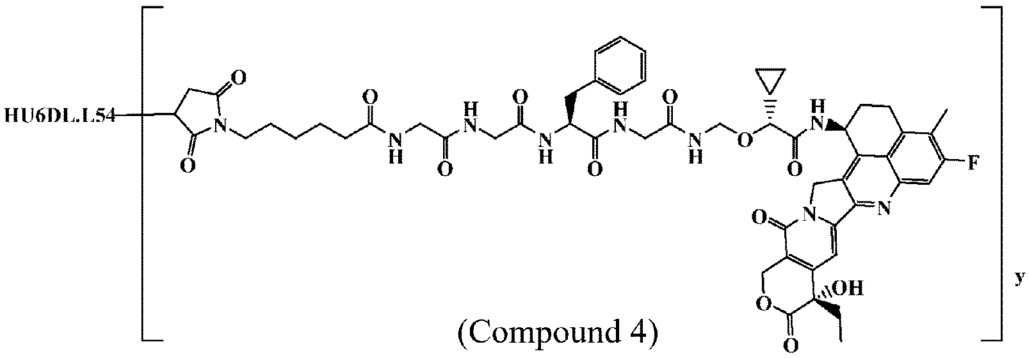

(Compound 4)

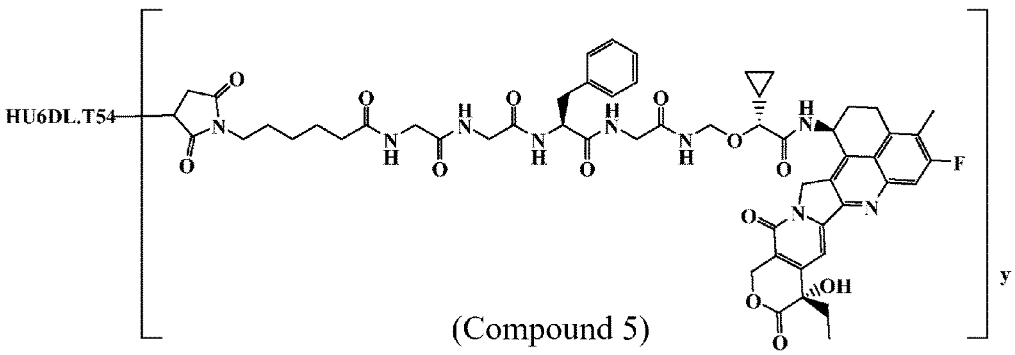

(Compound 5)

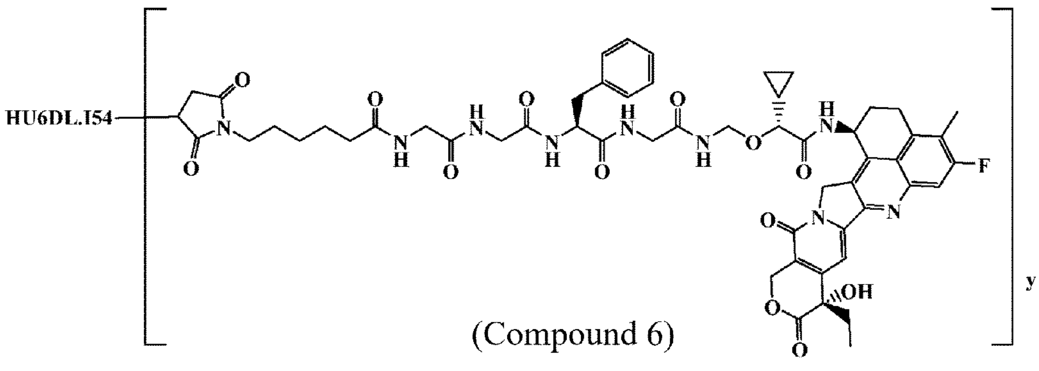

(Compound 6)

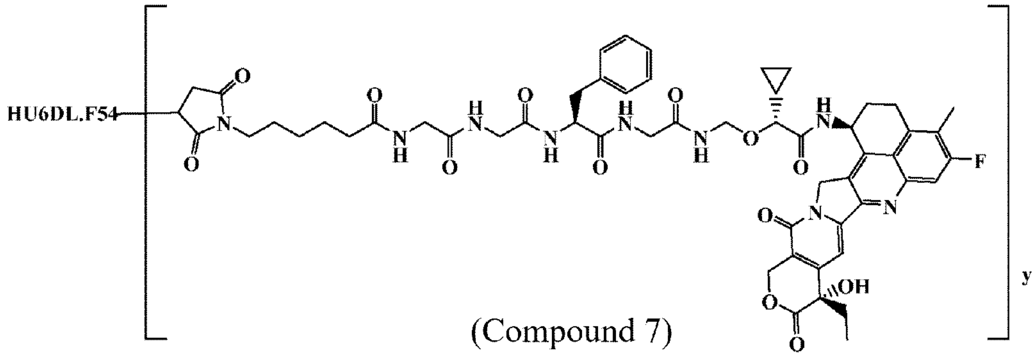

(Compound 7)

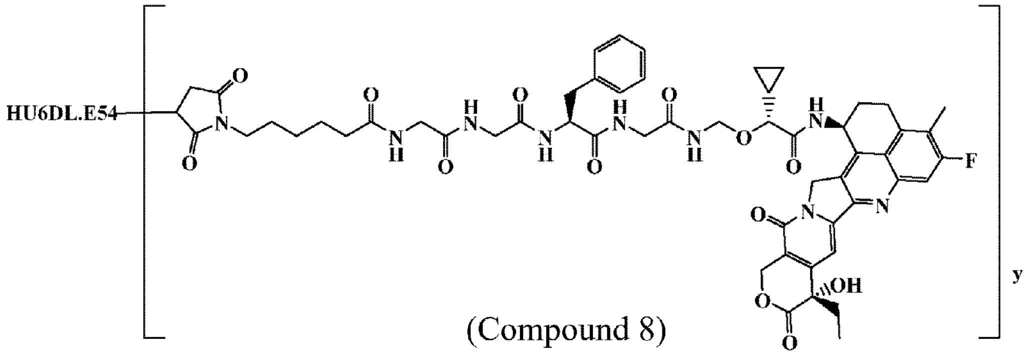

(Compound 8)

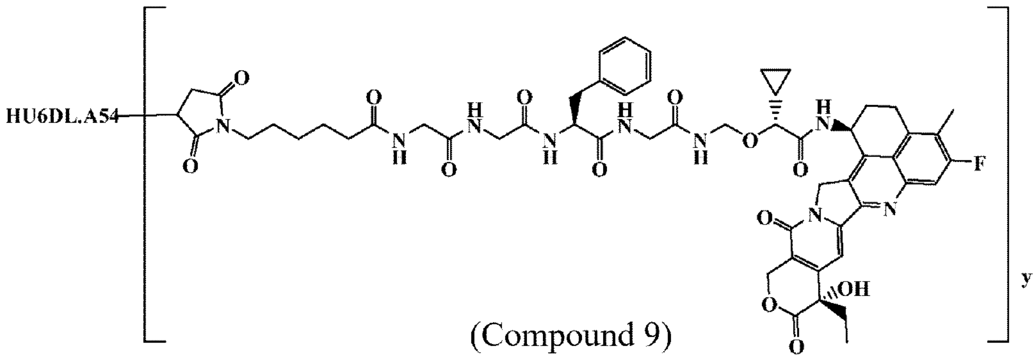

(Compound 9)

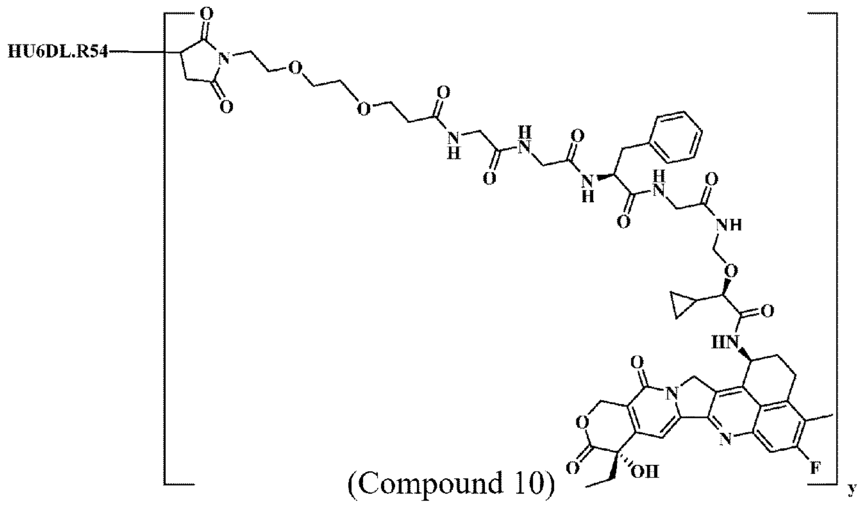

(Compound 10)

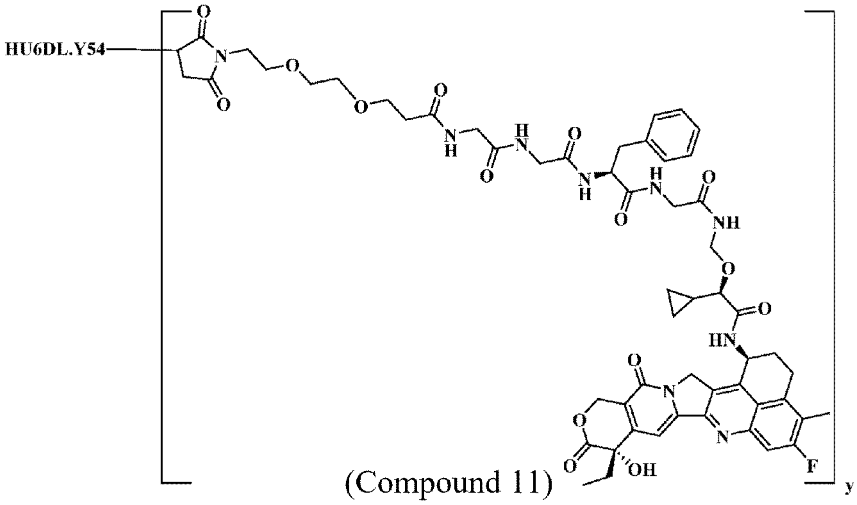

(Compound 11)

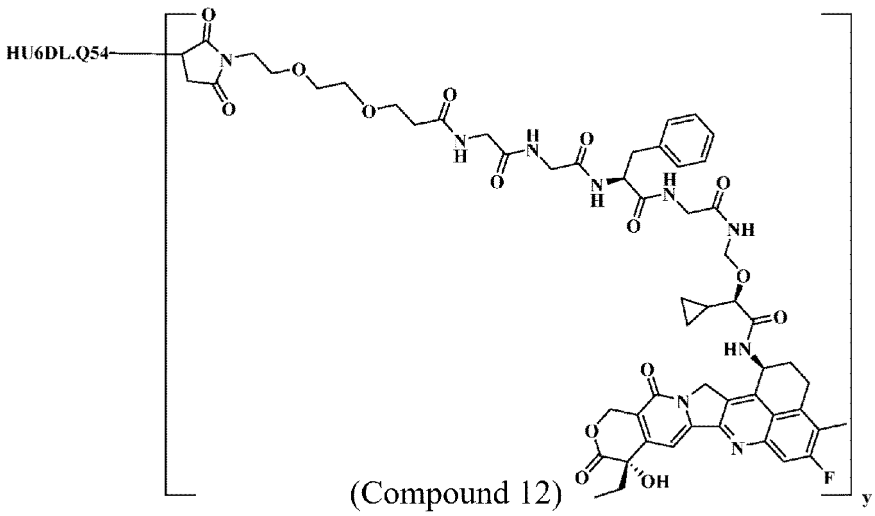

(Compound 12)

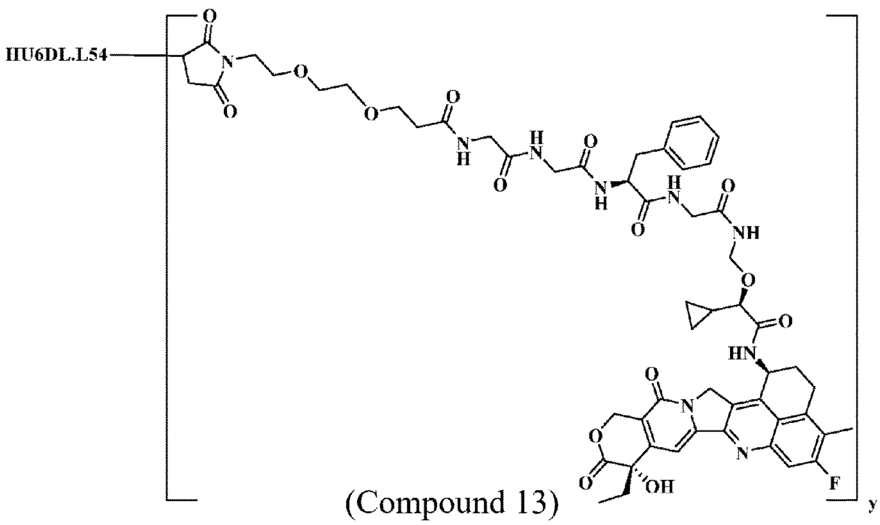

(Compound 13)

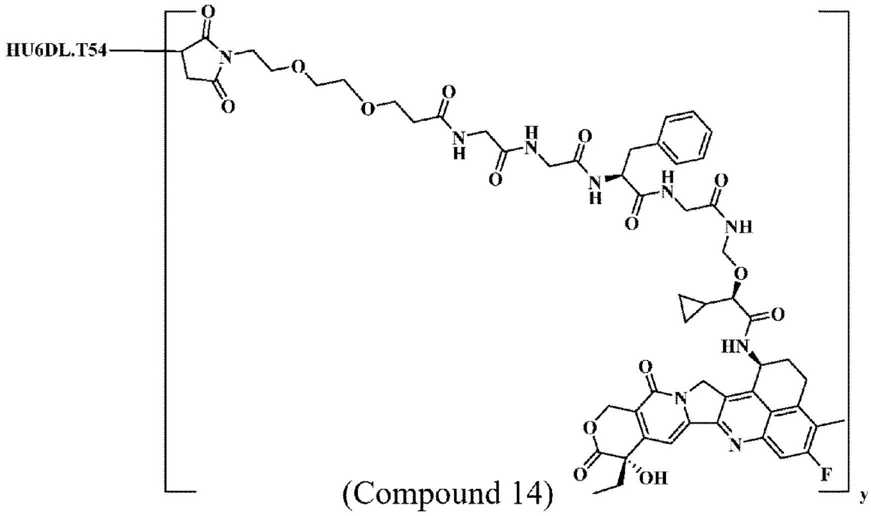

(Compound 14)

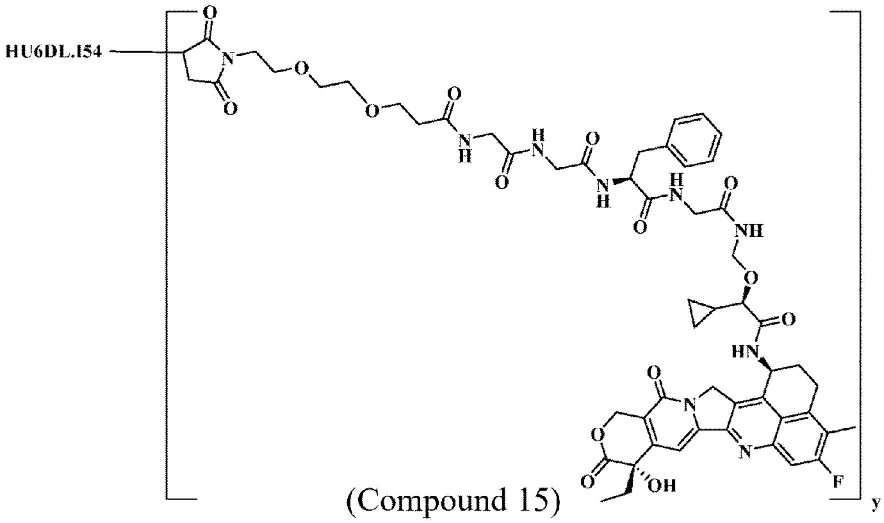

(Compound 15)

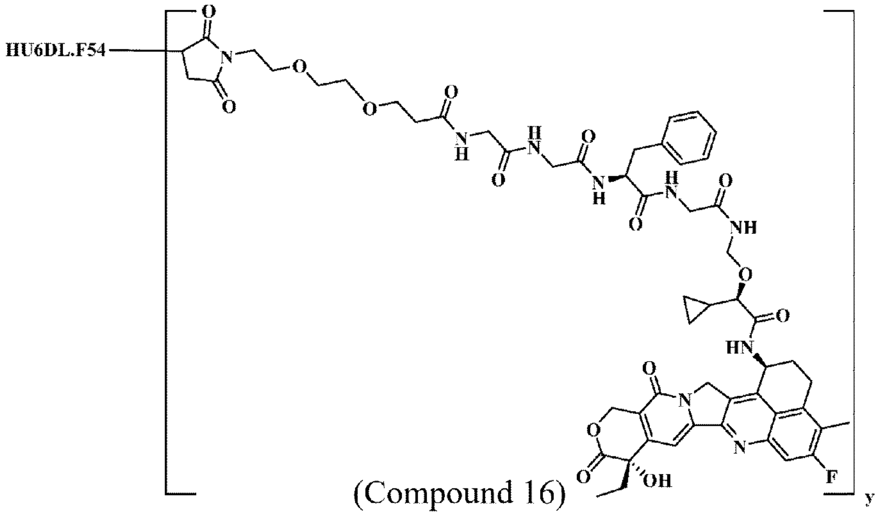

(Compound 16)

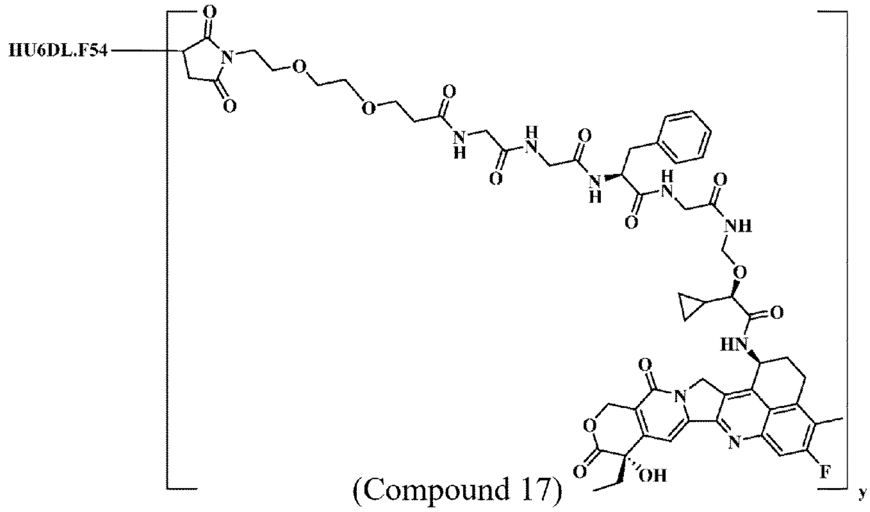

(Compound 17)

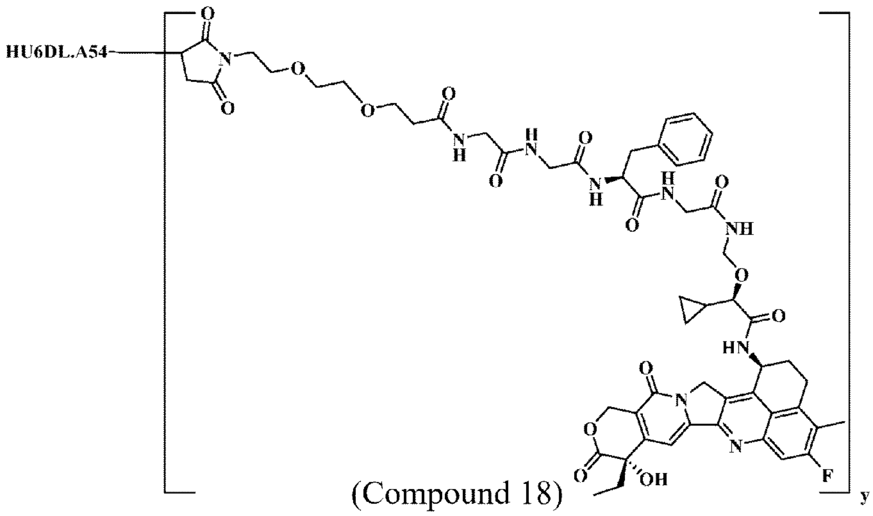

(Compound 18)

wherein y is as defined in claim 1.

The present invention also provides a method for preparing an antibody-drug conjugate of general formula (I) or a pharmaceutically acceptable salt or solvent compound, comprising the following steps:

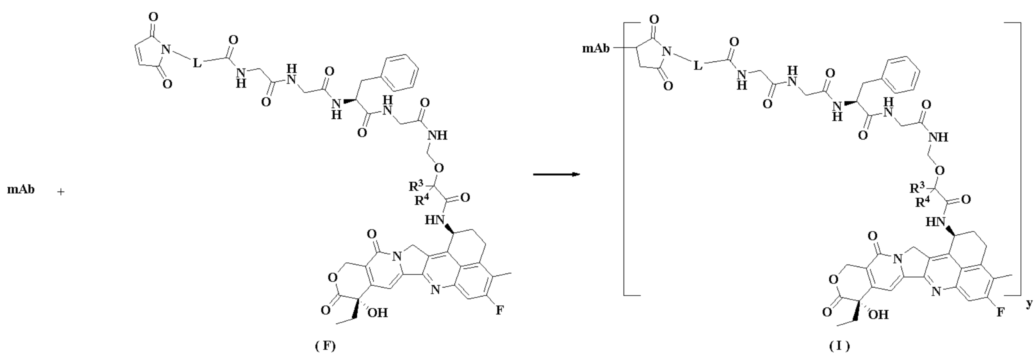

(F)

(I)

reducing a mAb, and then conjugating the reduced mAb with a compound of general formula (F) to obtain the compound of general formula (I);

wherein, L is as defined above.

mAb is selected from the group consisting of the above-mentioned anti-TROP-2 antibody or antigen binding fragment thereof;

y is 1-20; preferably 4-10; more preferably 4, 6, 8 or 10.

On another aspect, the present invention provides a pharmaceutical composition comprising the antibody-drug conjugate or the pharmaceutically acceptable salt or solvent compound thereof of the present invention, and one or more pharmaceutically acceptable excipients, diluents or carriers.

On another aspect, the present invention provides a pharmaceutical use. The present invention relates to the use of the anti-TROP-2 antibody-drug conjugate or the pharmaceutically acceptable salt or solvent compound of the antibody-drug conjugate or the pharmaceutical composition thereof for treating or preventing a disease or condition mediated by TROP-2.

On another aspect, the invention further provides the use of the antibody-drug conjugate of general formula (I) or the pharmaceutically acceptable salt or solvent compound of the antibody-drug conjugate or the pharmaceutical composition thereof in preparing a medicament for treating a human TROP-2 related disease.

In a more preferred embodiment of the present invention, the human TROP-2 related disease is a cancer with high expression of TROP-2, and the cancer is selected from the group consisting of triple-negative breast cancer, small Cellular lung cancer, urothelial carcinoma, human brain astroglioblastoma, human pharyngeal cancer, adrenal gland tumors, AIDS-related cancer, alveolar soft tissue sarcoma, astrocytoma, bladder cancer, bone cancer, brain and spinal cord cancer, metastatic brain tumors, breast cancer, carotid body tumors, cervical cancer, chondrosarcoma, chordoma, renal chromophobe cell carcinoma, clear cell carcinoma, colon cancer, colorectal cancer, desmoplastic small cell carcinoma Cellular neoplasm, ependymoma, Ewing tumor, extraskeletal myxoid chondrosarcoma, osteofibrous dysplasia, osteofibrous dysplasia, gallbladder or cholangiocarcinoma, gastric cancer, gestational trophoblastic disease, germ cell tumor, head and neck cancer, liver Cell carcinoma, islet cell tumor, Kaposin's sarcoma, renal cancer, leukemia, liposarcoma, malignant lipomatous tumor, liver cancer, lymphoma, lung cancer, medulloblastoma, melanoma, meningioma, multiple endocrine neoplasia, multiple myeloma, myelodysplastic syndrome, neuroblastoma, neuroendocrine tumors, ovarian cancer, pancreatic cancer, papillary thyroid cancer, parathyroidoma, pediatric cancer, peripheral nerve sheath tumor, phagocytoma, Pituitary tumors, prostate cancer, posterior uveal melanoma, renal metastasis cancer, rhabdoid tumor, rhabdomyosarcoma, sarcoma, skin cancer, soft tissue sarcoma, squamous cell carcinoma, synovial sarcoma, squamous cell carcinoma, thymic carcinoma, thymoma, metastatic thyroid cancer and uterine cancer.

The antibody-drug conjugate or the pharmaceutically acceptable salt or solvent compound thereof of the present invention can specifically bind to the target antigen, has high endocytosis efficiency, and a long half-life *in vivo,* and can significantly kill tumors while ensuring safety.

The antibody-drug conjugate and the pharmaceutically acceptable salt or solvent compound thereof of the present invention have significant anti-tumor effect and good safety, and also have good metabolic activity *in vivo,* long pharmaceutical effect *in vivo,* and broad clinical application prospects.

## Detailed Description of the present Invention

1. Terminology

In order that the present invention may be more readily understood, certain technical and scientific terms are specifically defined below. Unless otherwise clearly defined elsewhere in this document, all other technical and scientific terms used herein have the meaning commonly understood by one of ordinary skill in the art to which this invention belongs.

The three-letter and single-letter codes for amino acids used in the present invention are as described in J. Biol. Chem, 243, p3558 (1968).

The term "antibody" refers to an immunoglobulin, which is a tetrapeptide chain structure composed of two identical heavy chains and two identical light chains connected by inter-chain disulfide bonds. Since The amino acid composition and arrangement sequence of the heavy chain constant region of an immunoglobulin are different, the antigenicity of the immunoglobulin is also different. Accordingly, immunoglobulin can be divided into five classes, or isotypes of immunoglobulins, namely IgM, IgD, IgG, IgA and IgE, and the corresponding heavy chains thereof are a $\mu$ chain, a $\delta$ chain, a y chain, an $\alpha$ chain and an $\varepsilon$ chain, respectively. The same class of Ig can also be divided into different subclasses according to the differences in the amino acid composition of the hinge region thereof and the number and position of heavy chain disulfide bonds. For example, IgG can be divided into IgG1, IgG2, IgG3, and IgG4. The light chains are divided into a $\kappa$ chain or a $\lambda$ chain by the difference in the constant region. Each Ig class of the five Ig classes can have either a $\kappa$ chain or a $\lambda$ chain.

In the present invention, the antibody light chain variable region may further comprise a light chain constant region, and the light chain constant region includes a human or murine kappa, lambda chain or a variant thereof.

In the present invention, the antibody heavy chain variable region may further comprise a heavy chain constant region, and the heavy chain constant region includes a human or murine IgG1, IgG2, IgG3, IgG4 or a variant thereof.

The sequences of about 110 amino acids near the N-terminus of the antibody heavy chain and light chain vary greatly and are the variable regions (V regions); the rest of the amino acid sequence near the C-terminus is relatively stable and is the constant region (C region). The variable region includes 3 hypervariable regions (HVR) and 4 framework regions (FR) with relatively conserved sequences. Three hypervariable regions, also known as complementarity determining regions (CDRs), determine the specificity of the antibody. Each light chain variable region (VL) and heavy chain variable region (VH) consists of 3 CDR regions and 4 FR regions, which are arranged in the following order from amino end to carboxyl end: FR1, CDR1, FR2, CDR2, FR3, CDR3, FR4. The three CDR regions of the light chain refer to LCDR1, LCDR2, and LCDR3; the three CDR regions of the heavy chain refer to HCDR1, HCDR2 and HCDR3. The number and position of the CDR amino acid residues in the VL and VH regions of the antibody or the antigen binding fragment of the present invention conform to the known Kabat numbering rules and Kabat or ABM definition rules (http://bioinf.org.uk/abs/).

The term "TROP-2" includes any variant or isoform of TROP-2 that is naturally expressed by a cell. The antibody of the present invention may cross-react with TROP-2 from non-human species. Alternatively, the antibody may be specific for human TROP-2 and may not exhibit cross-reactivity with other species. TROP-2, or any variant or isoform thereof, can be isolated from the cells or tissues in which they are naturally expressed, or produced by recombinant techniques using techniques common in the art and described herein. Preferably, the anti-TROP-2 antibody targets human TROP-2 with a normal glycosylation pattern.

The term "recombinant human antibody" includes human antibodies prepared, expressed, created or isolated by recombinant methods, the techniques and methods involved are well known in the art, and the antibodies are such as:

1. Antibodies isolated from transgenic, transchromosomal animals (e.g., mice) with human immunoglobulin genes, or isolated from hybridomas prepared from transgenic, transchromosomal animals (e.g., mice) with human immunoglobulin genes;
2. Antibodies isolated from host cells, such as transfectomas, that have been transformed to express the antibodies;
3. Antibodies isolated from recombinant combinatorial human antibody libraries; and
4. Antibodies prepared, expressed, created or isolated by methods such as splicing human immunoglobulin gene sequences into other DNA sequences.

Such recombinant human antibodies include variable regions and constant regions that utilize specific human germline immunoglobulin sequences encoded by germline genes, but also include subsequent rearrangements and mutations such as those that occur during antibody maturation.

The term "murine antibody" in the present invention refers to a monoclonal antibody against human TROP-2 prepared according to the knowledge and skills in the art. Preparation involves injecting test subjects with TROP-2 antigen and then isolating hybridomas expressing antibodies with desired sequences or functional properties. In a preferred embodiment of the present invention, the murine TROP-2 antibody or the antigen binding fragment thereof may further comprise the light chain constant region of murine kappa, lambda chain or a variant thereof, or further comprise the heavy chain constant region of murine IgG1, IgG2, IgG3 or IgG4 or a variant thereof.

The term "human antibody" includes antibodies having variable and constant regions of human germline immunoglobulin sequences. Human antibodies of the present invention may include amino acid residues not encoded by human germline immunoglobulin sequences (for example, mutations introduced by random or site-specific mutagenesis *in vitro* or by somatic mutation *in vivo*). However, the term "human antibody" does not include antibodies in which CDR sequences derived from the germline of another mammalian species (such as mouse) have been grafted onto human backbone sequences (*i.e.*, "humanized antibodies").

The term "humanized antibody", also known as CDR-grafted antibody, refers to antibodies produced by grafting mouse CDR sequences into the human antibody variable region framework. Humanized antibodies can overcome the shortcomings of chimeric antibodies that induce a strong immune response due to carrying a large amount of mouse protein components. In order to avoid a decrease in activity while reducing immunogenicity, minimal reverse mutations can be performed on the human antibody variable region to maintain activity.

The term "chimeric antibody" refers to an antibody formed by fusing the variable region of a murine antibody with the constant region of a human antibody, which can reduce the immune response induced by the murine antibody. To create chimeric antibodies, it is needed to establish hybridomas that secrete specific murine monoclonal antibodies, then clone the variable region genes from mouse hybridoma cells, and then clone the constant region genes of human antibodies as needed, and link the mouse variable region genes with the human constant region gene to form a chimeric gene and then insert the chimeric gene into a human vector, and finally, express the chimeric antibody molecule in a eukaryotic industrial system or a prokaryotic industrial system. The constant region of the human antibody can be selected from the group consisting of the heavy chain constant region of human IgG1, IgG2, IgG3 or IgG4 or a variant thereof, preferably comprises the heavy chain constant region of human IgG1, IgG2 or IgG4, or the heavy chain constant region of IgG1 with amino acid mutations to enhance ADCC (anti-dependent cell-mediated cytotoxity) toxicity.

The term "antigen binding fragment" refers to an antigen binding fragment of an antibody and an antibody analog, which generally includes at least part of the antigen-binding region or variable region (for example, one or more CDRs) of the parent antibody. An antibody fragment retains at least some of the binding specificity of the parent antibody. Typically, the antibody fragment retains at least 10% of the binding activity of the parent antibody when the activity is expressed on a molar basis. Preferably, the antibody fragment retains at least 20%, 50%, 70%, 80%, 90%, 95% or 100% or more of the binding affinity of the parent antibody for the target. Examples of the antigen binding fragment include, but are not limited to: Fab, Fab', F(ab')2, a Fv fragment, a linear antibody, a single chain antibody, a nanobody, a domain antibody and a multispecific antibody. Engineered antibody variants are reviewed in Holliger and Hudson, 2005, Nat. Biotechnol. 23: 1126-1136.

The term "Fab fragment" consists of one light chain and the CH1 and variable region of one heavy chain. The heavy chain of a Fab molecule cannot form a disulfide bond with another heavy chain molecule.

The term "Fc" region includes the two heavy chain fragments comprising the CH2 and CH3 domains of an antibody. The two heavy chain fragments are held together by two or more disulfide bonds and by hydrophobic interactions of the CH3 domains.

The term "Fab' fragment" includes one light chain and a portion of a heavy chain that comprises the VH domain and the CH1 domain and the region between the CH1 and CH2 domains, whereby an interchain disulfide bond can be formed between the two heavy chains of two Fab' fragments to form a F(ab')2 molecule.

The term "F(ab')2 fragment" includes two light chains and two heavy chains comprising a part of the constant region between the CH1 and CH2 domains, whereby an interchain disulfide bond is formed between the two heavy chains. Therefore, the F(ab')2 fragment consists of two Fab' fragments held together by a disulfide bond between the two heavy chains.

The term "Fv region" includes variable regions from both heavy and light chains, but lacks constant regions.

The term "multispecific antibody" is used in its broadest sense to encompass antibodies with multi-epitope specificities. These multispecific antibodies include, but are not limited to: antibodies comprising a heavy chain variable region VH and a light chain variable region VL, wherein the VH-VL unit has multi-epitope specificity; antibodies with two or more VL and VH regions, each VH-VL unit binds to a different target or a different epitope of the same target; antibodies with two or more single variable regions, each single variable region binds to a different target or a different epitope of the same target; full-length antibodies, antibody fragments, diabodies, bispecific diabodies and triabodies, antibody fragments that have been covalently or non-covalently linked together, *etc.*

The term "single chain antibody" is a single-chain recombinant protein composed of the heavy chain variable region VH and the light chain variable region VL of an antibody connected through a connecting peptide, and is the smallest antibody fragment with a complete antigen-binding site.

The term "domain antibody fragment" is an immunoglobulin fragment with immunological function, which only contains a heavy chain variable region or a light chain variable region chain. In some cases, two or more VH regions are covalently linked to a peptide linker to form a bivalent domain antibody fragment. The two VH regions of the bivalent domain antibody fragment can target the same or different antigens.

The term "binds to TROP-2" refers to the ability to interact with human TROP-2.

The term "antigen-binding site" refers to a three-dimensional site recognized by the antibody or the antigen binding fragment of the present invention.

The term "epitope" refers to a site on an antigen to which an immunoglobulin or an antibody specifically binds. The epitope can be formed from adjacent amino acids, or non-adjacent amino acids that are juxtaposed by the tertiary folding of the protein. The epitope formed by adjacent amino acids are usually maintained after exposure to denaturing solvents, whereas the epitope formed by tertiary folding are usually lost after treatment with denaturing solvents. The epitope usually include at least 3-15 amino acids in a unique spatial conformation. Methods for determining what epitope is bound by a given antibody are well known in the art and include immunoblotting, immunoprecipitation assays, *etc.* Methods for determining the spatial conformation of an epitope include techniques in the art and techniques described herein, such as X-ray crystallography and two-dimensional nuclear magnetic resonance.

The terms "specific binding" and "selective binding" refer to the binding of an antibody to an epitope on a predetermined antigen. Typically, when the determination is carried out by surface plasmon resonance (SPR) technique using human TROP-2 as the analyte and antibody as the ligand in the instrument, the antibody binds to a predetermined antigen with an equilibrium dissociation constant ($K_D$) of about less than $10^{-7}$ M or even less, and the affinity of the antibody binding to the predetermined antigen is at least twice that of the antibody binding to a non-specific antigen (such as BSA) other than the predetermined antigen or a closely related antigen. The term "antibody that recognizes an antigen" is used interchangeably herein with the term "antibody that specifically binds to an antigen."

The term "cross-reactivity" refers to the ability of the antibody of the present invention to bind to TROP-2 from different species. For example, the antibody of the present invention that binds to human TROP-2 may also bind to TROP-2 of another species. Cross-reactivity is measured by detecting specific reactivity with a purified antigen in binding assays such as SPR and ELISA, or binding or functional interaction with cells physiologically expressing TROP-2.

Methods of determining cross-reactivity include standard binding assays as described herein, such as surface plasmon resonance (SPR) analysis, or flow cytometry.

The terms "inhibit" or "block" are used interchangeably and encompass both partial and complete inhibition/blocking. Inhibition/blocking of a ligand preferably reduces or alters the normal level or type of activity that would occur if ligand binding occurred without the inhibition or blocking. Inhibition and blocking are also intended to include any measurable reduction in ligand binding affinity when contacted with the anti-TROP-2 antibody compared to a ligand not contacted with the anti-TROP-2 antibody.

The term "growth inhibition" (for example, referring to a cell) is intended to include any measurable reduction in cell growth.

The terms "inducing an immune response" and "enhancing an immune response" are used interchangeably and refer to stimulation of an immune response to a specific antigen (*i.e.*, passive or adaptive). The term "induction" with respect to the induction of CDC or ADCC refers to the stimulation of specific direct cell killing mechanisms.

The term "ADCC" stands for antibody-dependent cell-mediated cytotoxicity, which means that cells expressing Fc receptors directly kill target cells coated by the antibody via recognizing the Fc segment of the antibody. The ADCC effector function of the antibody can be enhanced or reduced, reduced or eliminated by modifying the Fc segment of IgG. The modification refers to mutation in the heavy chain constant region of the antibody.

Methods for producing and purifying antibodies and antigen binding fragments are well known in the art and can be found in, for example, Cold Spring Harbor's Antibody Experimentation Technical Guide, Chapters 5-8 and 15. For example, mice can be immunized with human TROP-2 or a fragment thereof, and the resulting antibody can be renatured and purified, and sequenced for the amino acids using conventional methods. Antigen-binding fragments can also be prepared using conventional methods. In the antibody or the antigen binding fragment described in the present invention, one or more human FR regions are added to the CDR region of non-human origin by genetic engineering method. The human FR germline sequence can be obtained from the ImMunoGeneTics (IMGT) website at http://imgt.cines.fr, or from the Journal of Immunoglobulin, 2001ISBN012441351.

The engineered antibodies or the antigen binding fragments of the present invention can be prepared and purified using conventional methods. The cDNA sequence of the corresponding antibody can be cloned and recombined into the GS expression vector. The recombinant immunoglobulin expression vector can stably transfect CHO cells. As a more recommended prior art, mammalian expression systems result in glycosylation of the antibody, particularly at the highly conserved N-terminus of the FC region. Stable clones are obtained by expressing antibodies that specifically bind to human antigens. Positive clones were subjected to expanded culture in serum-free medium in a bioreactor to produce antibodies. The culture liquid containing secreted antibodies can be purified and collected using conventional techniques. Antibodies can be filtered and concentrated using conventional methods. Soluble mixtures and polymers can also be removed by conventional methods, such as molecular sieves and ion exchange. The obtained product needs to be frozen immediately, such as at -70°C, or freeze-dried.

Antibodies of the present invention refer to monoclonal antibodies. The monoclonal antibody (mAb) described in the present invention refers to an antibody obtained from a single clonal cell strain. The cell strain is not limited to eukaryotic, prokaryotic or phage clonal cell strains. Monoclonal antibodies or antigen binding fragments can be recombinantly obtained using hybridoma technology, recombinant technology, phage display technology, synthetic technology (such as CDR-grafting), or other existing technologies.

The terms "administer," "give," and "treat", when applied to animals, humans, experimental subjects, cells, tissues, organs, or biological fluids, refer to the contact of exogenous drugs, therapeutic agents, diagnostic agents or compositions with animals, humans, subjects, cells, tissues, organs or biological fluids. "Administer," "give," and "treat" may refer to, for example, therapeutic, pharmacokinetic, diagnostic, research and experimental procedures. Treatment of cells includes the contact of reagents with cells, and the contact of reagents with fluids, wherein the fluids are in contact with the cells. "Administer," "give," and "treat" also mean *in vitro* and *ex vivo* treatment of, for example, a cell by a reagent, a diagnostic composition or a binding composition or by another cell. "Treat" when applied to humans, veterinary subjects or research subjects refers to therapeutic treatment, prevention or prophylactic measures, and research and diagnostic applications.

The term "treat" means administering an therapeutic agent for internal or topical use, such as comprising any antibody of the present invention, to a patient having one or more symptoms of a disease for which the therapeutic agent is known to have a therapeutic effect. Generally, a therapeutic agent is administered in an amount effective to alleviate one or more symptoms of a disease in a treated patient or population, either by inducing regression of such symptoms or inhibiting the progression of such symptoms to any clinically measurable extent. The amount of a therapeutic agent that is effective in alleviating the symptoms of any particular disease (also referred to as a "therapeutically effective amount") can vary depending on a variety of factors, such as the patient's disease state, age and weight, and the ability of the drug to produce the desired therapeutic effect in the patient. Whether the symptoms of a disease have been alleviated may be assessed by any clinical testing method commonly used by physicians or other health care professionals to assess the severity or progression of the symptoms. Although embodiments of the present invention (e.g., treatments

or articles) may not be effective in alleviating the symptoms of a target disease in each patient, according to any statistical test method known in the art, such as Student t test, chi-square test, U test according to Mann and Whitney, Kruskal-Wallis test (H test), Jonckheere-Terpstra test and Wilcoxon test, the symptoms of the target disease should be alleviated in a statistically significant number of patients.

As used throughout the specification and claims, the term "consisting essentially of" or variations thereof is meant to include all stated elements or groups of elements, and optionally other elements of similar or different nature to the stated elements, which other elements do not significantly change the basic properties or new properties of the given dosage regimen, method or composition.

The term "naturally occurring" as applied to an object herein refers to the fact that the object is found in nature. For example, a polypeptide sequence or a polynucleotide sequence that exists in organisms (including viruses) that can be isolated from natural sources and has not been intentionally modified artificially in the laboratory is naturally occurring.

The term "effective amount" includes an amount sufficient to ameliorate or prevent symptoms of a medical condition or the condition. An effective amount also means an amount sufficient to allow or facilitate diagnosis. The effective amount for a particular patient or veterinary subject may vary depending on the following factors: for example, the condition to be treated, the patient's general health, the method, route and dosage of administration, and the severity of side effects. An effective amount may be the maximum dosage or dosage regimen that avoids significant side effects or toxic effects.

The term "exogenous" refers to substances that are produced outside organisms, cells or human bodies depending on the context.

The term "endogenous" refers to substances that are produced in cells, organisms, or human bodies depending on the context.

The term "homologous" refers to sequence similarity between two polynucleotide sequences or between two polypeptides. When a position in two compared sequences is occupied by the same base or amino acid monomer subunit, for example each position in two DNA molecules is occupied by adenine, then the molecules are homologous at that position. The percent homology between two sequences is a function of the number of matching or homologous positions shared by the two sequences divided by the number of positions compared $\times$ 100%. For example, when sequences are optimally aligned, if 6 out of 10 positions in two sequences match or are homologous, then the two sequences are 60% homologous. In general, comparisons are made when aligning two sequences yields the greatest percent homology.

The terms "cell," "cell line," and "cell culture" are used interchangeably, and all such designations include their progeny. Thus, the words "transformant" and "transformed cell" include primary subject cells and cultures derived therefrom, regardless of the number of transfers. It should also be understood that all progenies are unlikely to be exactly the same in terms of DNA content due to intentional or unintentional mutations. Mutant progenies that have the same function or biological activity as that screened in the originally transformed cells are included. "Optional" or "optionally" means that the event or circumstance subsequently described may but need not to occur, and the description includes the occasions where the events or circumstances occur or do not occur. For example, "optionally comprising 1-3 antibody heavy chain variable regions" means that an antibody heavy chain variable region of a specific sequence may, but need not, be present.

The term "pharmaceutical composition" means a composition containing one or more antibodies or antigen binding fragments thereof as described herein, and other ingredients such as physiologically/pharmaceutically acceptable carriers and excipients. The purpose of pharmaceutical compositions is to facilitate administration to living organisms and facilitate the absorption of active ingredients to exert biological activity.

The term "pharmaceutically acceptable salt" refers to a salt of the antibody-drug conjugate of the present invention, which are safe and effective when used in mammals, and have appropriate biological activity. The antibody-drug conjugate of the present invention contains at least one amino group and therefore can form a salt with an acid. Non-limiting examples of pharmaceutically acceptable salts include: hydrochloride, hydrobromide, hydroiodide, sulfate, disulfate, citrate, acetate, succinate, ascorbate, oxalate, nitrate, phosphate, hydrophosphate, dihydric phosphate, salicylate, hydrogen citrate, tartrate, maleate, fumarate, formate, benzoate, methanesulfonate, ethanesulfonate, benzene sulfonate, and p-toluenesulfonate.

The term "solvent compound" means that the antibody-drug conjugate compound of the present invention forms a pharmaceutically acceptable solvent compound with one or more solvent molecules, and non-limiting examples of the solvent molecules include: water, ethanol, acetonitrile, isopropyl alcohol, and ethyl acetate.

The term "cytotoxic drug" as used in the present invention refers to a substance that inhibits the function of a cell and/or causes cell death or destruction.

The term "tubulin inhibitor" refers to a class of compounds that interfere with the cell mitosis process by inhibiting the polymerization of tubulin or promoting the aggregation of tubulin, thereby exerting anti-tumor effects. Non-limiting examples include: maytansinoids, calicheamicins, taxanes, vincristine, colchicine, dolastatin/auristatin/monomethyl auristatin E (MMAE)/monomethyl auristatin F(MMAF).

The term "linker" refers to a chemical module containing a covalent bond or chain of atoms that covalently attaches the antibody to the drug. Non-limiting examples of linkers include: arylene, heteroarylene, PEG, polymethyleneoxy, succinate, succinamide, diglycolate, malonate and caproamide.

The term "drug load" (also referring to drug-to-antibody ratio (DAR)) is represented by y, the average number of cytotoxic drugs per antibody in general formula (A). The drug load in the present invention can range from 1 to 20 cytotoxic drugs (D) per antibody. The antibody-drug conjugate of general formula (A) is a collection of antibodies conjugated with a certain range (1-20) of cytotoxic drugs. The drug load (DAR) in the antibody-drug conjugate from the conjugation reaction can be characterized by conventional means, such as mass spectrometry, HPLC, and ELISA. The quantitative distribution of antibody-drug conjugates in y-valuescan be determined by these means.

The present invention further includes various deuterated forms of the compound of formula (I). Each available hydrogen atom attached to a carbon atom can be independently replaced by a deuterium atom. Those skilled in the art can synthesize deuterated forms of the compound of formula (I) by referring to relevant literatures. The deuterated forms of the compound of formula (I) can be prepared using commercially available deuterated starting materials, or can be synthesized using deuterated reagents using conventional techniques. The deuterated reagents include, but are not limited to, deuterated borane, solution of trideuterated borane in tetrahydrofuran, deuterated lithium aluminum hydride, deuterated iodoethane and deuterated iodomethane.

In some embodiments of the present invention, the cytotoxic drug is conjugated to the N-terminal amino group and/or the epsilon-amino group of the lysine residue of the ligand through a linking unit. In other embodiments of the present invention, the cytotoxic drug is conjugated to sulfhydryl group of the ligand through a linking unit. Generally, the number of drug molecules that can be conjugated to the antibody in the conjugation reaction would be less than the theoretical maximum.

The following non-limiting methods can be used to control the loading of ligand-cytotoxic drug conjugate and include:

(1) Controlling the molar ratio of linking reagent to monoclonal antibody,
(2) Controlling reaction time and temperature,
(3) Choosing different reaction reagents.

The antibody-drug conjugate or the pharmaceutically acceptable salt or solvent compound thereof of the present invention has significant anti-tumor effect and good safety.

**BRIEF DESCRIPTION OF THE DRAWINGS**

Fig. 1 is the $^1$H-NMR spectrum of compound D.

**DETAILED DESCRIPTION OF THE INVENTION**

The following examples are used to further describe the present invention, but these examples do not limit the scope of the present invention. Experimental methods without specifying specific conditions in the examples of the present invention usually follow conventional conditions, such as in Cold Spring Harbor's Antibody Technology Experiment Manual and Molecular Cloning Manual; or follow the conditions recommended by the raw material or product manufacturer. The reagents for which the specific source is not indicated, are conventional commercially available reagents.

**Example 1: HU6DL variant design experiment**

The CDR sequences of the humanized antibody HU6DL heavy chain and light chain disclosed in patent WO 2020228604 are shown in Table 1 below. Because of the motif $N^{54}D^{55}S^{56}$ in HCDR2, glycosylation is easy to occur.

Table 1. CDR sequences of heavy chain and light chain variable regions of humanized antibody HU6DL

| Name | Sequence | Number |
|---|---|---|
| HCDR1 | NYWNIN | SEQ ID NO: 3 |
| HCDR2 | RIDPNDSETHYNQKFKD | SEQ ID NO: 4 |
| HCDR3 | SGFGSTYWFFDV | SEQ ID NO: 5 |

(continued)

| Name | Sequence | Number |
|---|---|---|
| LCDR1 | KASQDVSTAVA | SEQ ID NO: 6 |
| LCDR2 | SASYRYT | SEQ ID NO: 7 |
| LCDR3 | QQHYSTPLT | SEQ ID NO: 8 |

The mutants HU6DL.R54, HU6DL.Y54, HU6DL.Q54, HU6DL.L54HU6DL.T54, HU6DL.I54, HU6DL.F54, HU6DL.E54 and HU6DL.A54 of antibody HU6DL were obtained by site-directed mutation of N54 with computer-aided technology which reduced the potential glycosylation risk without affecting its binding to antigen and its own thermal stability. The sequence of the HCDR2 of the corresponding heavy chain was as follows:

Table 2. Heavy chain variable region HCDR2 sequences of HU6DL mutants

| Name | Sequence | Number |
|---|---|---|
| HU6DL.R54 | RIDPRD SETHYNQKFKD | SEQ ID NO: 9 |
| HU6DL.Y54 | RIDPYDSETHYNQKFKD | SEQ ID NO: 10 |
| HU6DL.Q54 | RIDPQDSETHYNQKFKD | SEQ ID NO: 11 |
| HU6DL.L54 | RIDPLDSETHYNQKFKD | SEQ ID NO: 12 |
| HU6DL.T54 | RIDPTDSETHYNQKFKD | SEQ ID NO: 13 |
| HU6DL.I54 | RIDPIDSETHYNQKFKD | SEQ ID NO: 14 |
| HU6DL.F54 | RIDPFDSETHYNQKFKD | SEQ ID NO: 15 |
| HU6DL.E54 | RIDPEDSETHYNQKFKD | SEQ ID NO: 16 |
| HU6DL.A54 | RIDPADSETHYNQKFKD | SEQ ID NO: 17 |

The corresponding heavy chain and light chain variable regions were as follows:

HU6DL.R54 HCVR

EVQLLESGGGLVQPGGSLRLSCAASGFTVSNYWMNWVRQAPGKGLEWMGRIDPRD
SETHYNQKFKDRVTISVDKSKNQFSLKLSSVTAADTAVYYCARSGFGSTYWFFDVWG
QGTTVTVSS
SEQ ID NO: 18

HU6DL.R54 LCVR

DVVMTQSPLSLPVTLGQPASISCKASQDVSTAVAWYQQKPGKAPKLFIYSASYRYTG
VPSRFSGSGSGTDFTLTISSLQPEDFATYYCQQHYSTPLTFGQGTRLEIK
SEQ ID NO: 19

HU6DL.Y54 HCVR

EVQLLESGGGLVQPGGSLRLSCAASGFTVSNYWMNWVRQAPGKGLEWMGRIDPYD
SETHYNQKFKDRVTISVDKSKNQFSLKLSSVTAADTAVYYCARSGFGSTYWFFDVWG
QGTTVTVSS
SEQ ID NO: 20

HU6DL.Y54 LCVR

DVVMTQSPLSLPVTLGQPASISCKASQDVSTAVAWYQQKPGKAPKLFIYSASYRYTG
VPSRFSGSGSGTDFTLTISSLQPEDFATYYCQQHYSTPLTFGQGTRLEIK
SEQ ID NO: 19

HU6DL.Q54 HCVR

EVQLLESGGGLVQPGGSLRLSCAASGFTVSNYWMNWVRQAPGKGLEWMGRIDPQD
SETHYNQKFKDRVTISVDKSKNQFSLKLSSVTAADTAVYYCARSGFGSTYWFFDVW
GQGTTVTVSS
SEQ ID NO: 21

HU6DL.Q54 LCVR

DVVMTQSPLSLPVTLGQPASISCKASQDVSTAVAWYQQKPGKAPKLFIYSASYRYTGV
PSRFSGSGSGTDFTLTISSLQPEDFATYYCQQHYSTPLTFGQGTRLEIK
SEQ ID NO: 19

HU6DL.L54 HCVR

EVQLLESGGGLVQPGGSLRLSCAASGFTVSNYWMNWVRQAPGKGLEWMGRIDPLD
SETHYNQKFKDRVTISVDKSKNQFSLKLSSVTAADTAVYYCARSGFGSTYWFFDVW
GQGTTVTVSS
SEQ ID NO: 22

HU6DL.L54 LCVR

DVVMTQSPLSLPVTLGQPASISCKASQDVSTAVAWYQQKPGKAPKLFIYSASYRYTG
VPSRFSGSGSGTDFTLTISSLQPEDFATYYCQQHYSTPLTFGQGTRLEIK
SEQ ID NO: 19

HU6DL.T54 HCVR

EVQLLESGGGLVQPGGSLRLSCAASGFTVSNYWMNWVRQAPGKGLEWMGRIDPTD
SETHYNQKFKDRVTISVDKSKNQFSLKLSSVTAADTAVYYCARSGFGSTYWFFDVWG
QGTTVTVSS
SEQ ID NO: 23

HU6DL.T54 LCVR

DVVMTQSPLSLPVTLGQPASISCKASQDVSTAVAWYQQKPGKAPKLFIYSASYRYTG
VPSRFSGSGSGTDFTLTISSLQPEDFATYYCQQHYSTPLTFGQGTRLEIK
SEQ ID NO: 19

HU6DL.I54 HCVR

EVQLLESGGGLVQPGGSLRLSCAASGFTVSNYWMNWVRQAPGKGLEWMGRIDPIDS
ETHYNQKFKDRVTISVDKSKNQFSLKLSSVTAADTAVYYCARSGFGSTYWFFDVWG
QGTTVTVSS
SEQ ID NO: 24

HU6DL.I54 LCVR

DVVMTQSPLSLPVTLGQPASISCKASQDVSTAVAWYQQKPGKAPKLFIYSASYRYTG
VPSRFSGSGSGTDFTLTISSLQPEDFATYYCQQHYSTPLTFGQGTRLEIK
SEQ ID NO: 19

HU6DL.F54 HCVR

EVQLLESGGGLVQPGGSLRLSCAASGFTVSNYWMNWVRQAPGKGLEWMGRIDPFDS
ETHYNQKFKDRVTISVDKSKNQFSLKLSSVTAADTAVYYCARSGFGSTYWFFDVWG
QGTTVTVSS

SEQ ID NO: 25

HU6DL.F54 LCVR

DVVMTQSPLSLPVTLGQPASISCKASQDVSTAVAWYQQKPGKAPKLFIYSASYRYTG
VPSRFSGSGSGTDFTLTISSLQPEDFATYYCQQHYSTPLTFGQGTRLEIK
SEQ ID NO: 19

HU6DL.E54 HCVR

EVQLLESGGGLVQPGGSLRLSCAASGFTVSNYWMNWVRQAPGKGLEWMGRIDPEDS
ETHYNQKFKDRVTISVDKSKNQFSLKLSSVTAADTAVYYCARSGFGSTYWFFDVWG
QGTTVTVSS
SEQ ID NO: 26

HU6DL.E54 LCVR

DVVMTQSPLSLPVTLGQPASISCKASQDVSTAVAWYQQKPGKAPKLFIYSASYRYTG
VPSRFSGSGSGTDFTLTISSLQPEDFATYYCQQHYSTPLTFGQGTRLEIK
SEQ ID NO: 19

HU6DL.A54 HCVR

EVQLLESGGGLVQPGGSLRLSCAASGFTVSNYWMNWVRQAPGKGLEWMGRIDPAD
SETHYNQKFKDRVTISVDKSKNQFSLKLSSVTAADTAVYYCARSGFGSTYWFFDVWG
QGTTVTVSS
SEQ ID NO: 27

HU6DL.A54 LCVR

DVVMTQSPLSLPVTLGQPASISCKASQDVSTAVAWYQQKPGKAPKLFIYSASYRYTG
VPSRFSGSGSGTDFTLTISSLQPEDFATYYCQQHYSTPLTFGQGTRLEIK
SEQ ID NO: 19

The designed sequences of the heavy chain and light chain variable regions are connected to the sequences of the IgG1 heavy chain constant region and light chain constant region respectively. The connected human IgG1 heavy chain constant region sequence was as follows:
IgG1 C1

ASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKKVEPKSCDKTHTCPPCPAPELL
GGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYT
LPPSRDELTKNQVSLTCLVKGFYPSDIAVEWESNGQPENNYKTTPPVLDSDGSFFLYS
KLTVDKSRWQQGNVFSCSVMHEALHNHYTQKSLSLSPGK
SEQ ID NO: 1

The connected human kappa chain constant region sequence was as follows:
Ig kappa C

RTVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
SEQ ID NO: 2

After connection, the resulting exemplary heavy and light chain sequences were as follows:

HU6DL.R54 HC

EVQLLESGGGLVQPGGSLRLSCAASGFTVSNYWMNWVRQAPGKGLEWMGRIDPRDS
ETHYNQKFKDRVTISVDKSKNQFSLKLSSVTAADTAVYYCARSGFGSTYWFFDVWGQ
GTTVTVSSASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCDKTHTCP
PCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQP
REPQVYTLPPSRDELTKNQVSLTCLVKGFYPSDIAVEWESNGQPENNYKTTPPVLDSDG
SFFLYSKLTVDKSRWQQGNVFSCSVMHEALHNHYTQKSLSLSPGK
SEQ ID NO: 28

HU6DL.R54 LC

DVVMTQSPLSLPVTLGQPASISCKASQDVSTAVAWYQQKPGKAPKLFIYSASYRYTG
VPSRFSGSGSGTDFTLTISSLQPEDFATYYCQQHYSTPLTFGQGTRLEIKRTVAAPSVFIF
PPSDEQLKSGTASVVCLLNNFYPREAKVQWKVDNALQSGNSQESVTEQDSKDSTYSLS
STLTLSKADYEKHKVYACEVTHQGLSSPVTKSFNRGEC
SEQ ID NO: 29

HU6DL.Y54 HC

EVQLLESGGGLVQPGGSLRLSCAASGFTVSNYWMNWVRQAPGKGLEWMGRIDPYD
SETHYNQKFKDRVTISVDKSKNQFSLKLSSVTAADTAVYYCARSGFGSTYWFFDVWG
QGTTVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTH
TCPPCPAPELLGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKTTPPV
LDSDGSFFLYSKLTVDKSRWQQGNVFSCSVMHEALHNHYTQKSLSLSPGK
SEQ ID NO: 30

HU6DL.Y54 LC

DVVMTQSPLSLPVTLGQPASISCKASQDVSTAVAWYQQKPGKAPKLFIYSASYRYTG
VPSRFSGSGSGTDFTLTISSLQPEDFATYYCQQHYSTPLTFGQGTRLEIKRTVAAPSVFIF
PPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC
SEQ ID NO: 29

HU6DL.Q54 HC

EVQLLESGGGLVQPGGSLRLSCAASGFTVSNYWMNWVRQAPGKGLEWMGRIDPQD
SETHYNQKFKDRVTISVDKSKNQFSLKLSSVTAADTAVYYCARSGFGSTYWFFDVWG
QGTTVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAKG
QPREPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK
SEQ ID NO: 31

HU6DL.Q54 LC

DVVMTQSPLSLPVTLGQPASISCKASQDVSTAVAWYQQKPGKAPKLFIYSASYRYTG
VPSRFSGSGSGTDFTLTISSLQPEDFATYYCQQHYSTPLTFGQGTRLEIKRTVAAPSVFIF
PPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC
SEQ ID NO: 29

HU6DL.L54 HC

EVQLLESGGGLVQPGGSLRLSCAASGFTVSNYWMNWVRQAPGKGLEWMGRIDPLDS
ETHYNQKFKDRVTISVDKSKNQFSLKLSSVTAADTAVYYCARSGFGSTYWFFDVWGQ
GTTVTVSSASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCDKTHTCP
PCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQP
REPQVYTLPPSRDELTKNQVSLTCLVKGFYPSDIAVEWESNGQPENNYKTTPPVLDSDG
SFFLYSKLTVDKSRWQQGNVFSCSVMHEALHNHYTQKSLSLSPGK
SEQ ID NO: 32

HU6DL.L54 LC

DVVMTQSPLSLPVTLGQPASISCKASQDVSTAVAWYQQKPGKAPKLFIYSASYRYTG
VPSRFSGSGSGTDFTLTISSLQPEDFATYYCQQHYSTPLTFGQGTRLEIKRTVAAPSVFIF

PPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC
SEQ ID NO: 29

HU6DL.T54 HC

EVQLLESGGGLVQPGGSLRLSCAASGFTVSNYWMNWVRQAPGKGLEWMGRIDPTDS
ETHYNQKFKDRVTISVDKSKNQFSLKLSSVTAADTAVYYCARSGFGSTYWFFDVWG
QGTTVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAKG
QPREPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK
SEQ ID NO: 33

HU6DL.T54 LC

DVVMTQSPLSLPVTLGQPASISCKASQDVSTAVAWYQQKPGKAPKLFIYSASYRYTG
VPSRFSGSGSGTDFTLTISSLQPEDFATYYCQQHYSTPLTFGQGTRLEIKRTVAAPSVFIF
PPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC
SEQ ID NO: 29

HU6DL.I54 HC

EVQLLESGGGLVQPGGSLRLSCAASGFTVSNYWMNWVRQAPGKGLEWMGRIDPIDS
ETHYNQKFKDRVTISVDKSKNQFSLKLSSVTAADTAVYYCARSGFGSTYWFFDVWGQ
GTTVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAKG
QPREPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK
SEQ ID NO: 34

HU6DL.I54 LC

DVVMTQSPLSLPVTLGQPASISCKASQDVSTAVAWYQQKPGKAPKLFIYSASYRYTG
VPSRFSGSGSGTDFTLTISSLQPEDFATYYCQQHYSTPLTFGQGTRLEIKRTVAAPSVFIF
PPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC
SEQ ID NO: 29

HU6DL.F54 HC

EVQLLESGGGLVQPGGSLRLSCAASGFTVSNYWMNWVRQAPGKGLEWMGRIDPFDS
ETHYNQKFKDRVTISVDKSKNQFSLKLSSVTAADTAVYYCARSGFGSTYWFFDVWG
QGTTVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTH
TCPPCPAPELLGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAK
GQPREPQVYTLPPSRDELTKNQVSLTCLVKGFYPSDIAVEWESNGQPENNYKTTPPVL
DSDGSFFLYSKLTVDKSRWQQGNVFSCSVMHEALHNHYTQKSLSLSPGK
SEQ ID NO: 35

HU6DL.F54 LC

DVVMTQSPLSLPVTLGQPASISCKASQDVSTAVAWYQQKPGKAPKLFIYSASYRYTG
VPSRFSGSGSGTDFTLTISSLQPEDFATYYCQQHYSTPLTFGQGTRLEIKRTVAAPSVFIF
PPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC
SEQ ID NO: 29

HU6DL.E54 HC

EVQLLESGGGLVQPGGSLRLSCAASGFTVSNYWMNWVRQAPGKGLEWMGRIDPEDS
ETHYNQKFKDRVTISVDKSKNQFSLKLSSVTAADTAVYYCARSGFGSTYWFFDVWG
QGTTVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTH
TCPPCPAPELLGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKTTPPV
LDSDGSFFLYSKLTVDKSRWQQGNVFSCSVMHEALHNHYTQKSLSLSPGK
SEQ ID NO: 36

HU6DL.E54 LC

DVVMTQSPLSLPVTLGQPASISCKASQDVSTAVAWYQQKPGKAPKLFIYSASYRYTG
VPSRFSGSGSGTDFTLTISSLQPEDFATYYCQQHYSTPLTFGQGTRLEIKRTVAAPSVFIF
PPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC
SEQ ID NO: 29

HU6DL.A54 HC

EVQLLESGGGLVQPGGSLRLSCAASGFTVSNYWMNWVRQAPGKGLEWMGRIDPAD
SETHYNQKFKDRVTISVDKSKNQFSLKLSSVTAADTAVYYCARSGFGSTYWFFDVWG

QGTTVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTH
TCPPCPAPELLGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAK
GQPREPQVYTLPPSRDELTKNQVSLTCLVKGFYPSDIAVEWESNGQPENNYKTTPPVL
DSDGSFFLYSKLTVDKSRWQQGNVFSCSVMHEALHNHYTQKSLSLSPGK
SEQ ID NO: 37

HU6DL.A54 LC

DVVMTQSPLSLPVTLGQPASISCKASQDVSTAVAWYQQKPGKAPKLFIYSASYRYTG
VPSRFSGSGSGTDFTLTISSLQPEDFATYYCQQHYSTPLTFGQGTRLEIKRTVAAPSVFIF
PPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC
SEQ ID NO: 29

cDNA fragments were synthesized based on the amino acid sequences of the light and heavy chains of each humanized antibody above, and HU6DL protein mutants were expressed by means of transient transfection of HEK293 cells, and the purity of the antibody was detected by molecular exclusion chromatography. The concentration and purity were shown in Table 3 below.

Table 3. Concentration and purity of HU6DL mutants

| Name | Concentration (mg/mL) | Purity (%) |
|---|---|---|
| HU6DL.R54 | 1.99 | 95.8 |
| HU6DL.Y54 | 1.34 | 96.4 |
| HU6DL.Q54 | 1.73 | 93.1 |
| HU6DL.L54 | 1.36 | 94.9 |
| HU6DL.T54 | 2.73 | 96.1 |
| HU6DL.I54 | 2.00 | 95.5 |
| HU6DL.F54 | 1.68 | 93.5 |
| HU6DL.E54 | 1.56 | 95.6 |
| HU6DL.A54 | 1.00 | 95.1 |

**Example 2: Eexperiment on the affinity of HU6DL mutants for Trop-2 antigen**

Experiment purpose:

The ELISA sandwich method, namely "antigen-antibody-HRP labeled secondary antibody", was used to evaluate the differences in the affinity levels of different anti-Trop-2 mutants for Trop-2 antigen.

Experimental steps:

Trop-2 protein (his tag) (SinoBiologics, Cat: 10428-H08H) was diluted to 1 ug/mL by DPBS with pH 7.4, and the diluted solution was added into a high affinity 96-well plate (Corning, Cat: 3590) at 100 μL/well, and the mixture was incubated overnight at 4°C. The next day, the Trop-2 antigen solution was spun off, and a solution containing 0.05% Tween 20 PBS pH7.4 (PBST) was added at 200 μL/well, washing was performed 3 times, and then 2% BSA (dissolved in PBST) was added at 200 μL/well, blocking was performed at 37°C for 1 h, and PBST was used for washing 3 times. Candidate antibody serially diluted by 10-fold for 8 concentration gradients (initial concentration was 10 nM-1 $\times 10^{-6}$ nM) was added at 100 μL/well, 0.5% BSA was used as negative control, blocking was performed at 37°C for 1 h, and PBST was used for washing 3 times, the solution of goat anti-human IgG Fc-HRP (abcam, cat: ab97225) secondary antibody diluted at 1 : 10000 was added at 100 μL/ well, blocking was performed at 37°C for 1 h, and PBST was used for washing 3 times.

TMB (CST, Cat: 7004P6) substrate was added at 100 μL/ well, and the mixture was reacted at room temperature for 3 min until the color of the well containing the solution with the highest antibody concentration turned dark blue. Termination solution (CST, Cat: 7002P6) was added at 50 μL/ well to terminate the reaction, and the absorbance (OD) was read at 450 nm.

Data processing:

The $EC_{50}$ of the affinity of each HU6DL mutant for the antigen was calculated by the GraphPad PRISM 8.0 log(agonist) vs. response-viable slope (four parameters) formula taking the logarithmic value of the candidate antibody concentration as the X-axis coordinate and the absorbance value of OD450 as the ordinate. The affinity of the HU6DL mutants for human TROP-2 antigen ($EC_{50}$) was shown in Table 4 below:

Table 4 Affinity of HU6DL mutants for human TROP-2 antigen ($EC_{50}$)

| Name | Top OD450 | Affinity $EC_{50}$ (nM) |
|---|---|---|
| HU6DL.R54 | 2.05 | 0.052 |
| HU6DL.Y54 | 1.93 | 0.051 |
| HU6DL.Q54 | 1.91 | 0.086 |
| HU6DL.L54 | 1.96 | 0.048 |
| HU6DL.T54 | 1.90 | 0.052 |
| HU6DL.I54 | 1.96 | 0.063 |
| HU6DL.F54 | 1.93 | 0.067 |
| HU6DL.E54 | 1.96 | 0.064 |
| HU6DL.A54 | 2.07 | 0.062 |

Experimental conclusion:

The above data showed that the HU6DL mutants of the present invention had good affinity for the human TROP-2 antigen.

**Example 3: Eexperiment on the affinity of HU6DL mutants for tumor cells**

Experiment purpose:

Flow cytometry was used to evaluate the differences in affinity levels of HU6DL mutants for tumor cell lines expressing Trop-2 antigen.

Experimental reagents:

Gastric cancer cell NCI-N87 (purchased from the Cell Bank of the Chinese Academy of Sciences, TCHu130);
Non-small cell lung cancer cell HCC827 (purchased from the Cell Bank of the Chinese Academy of Sciences, TCHu153);
Bladder cancer cell SW780 (purchased from the Cell Bank of the Chinese Academy of Sciences, TCHu219);
Bladder cancer cell RT4 (purchased from the Cell Bank of the Chinese Academy of Sciences, TCHu226);

Experimental steps:

Accutase (*Sigma, cat: A6964*) digestion solution was used to treat tumor cells in good growth status, a single cell suspension was prepared with 2% FBS (diluted in DBPS, pH 7.4) solution, and the cell density was adjusted to 1 $\times$ $10^6$ cells/mL. The suspension was evenly distributed to 96-well V-shaped bottom plate at 100 $\mu$L/ well, centrifuged at 4°C at 300 g $\times$ 5 min, and the supernatant was discarded. Solutions of candidate antibody serially diluted by 10-fold for 10 concentration gradients (1000 nM-1 $\times$ $10^{-6}$ nM) were added at 100 $\mu$L/well, and the mixture was incubated at 4°C for 1 h,
centrifuged at 4°C at 300 g $\times$ 5 min, and washed twice. A solution of mouse anti-human IgG Fc secondary antibody labeled with PE (*Biolegend, cat: 409304*) diluted at the ratio of 5 $\mu$L/$10^6$ cells was added at 100 $\mu$L/well, and the mixture was incubated at 4°C for 1h, centrifuged at 4°C at 300 g $\times$ 5 min, and washed twice. 70 $\mu$L of 2% FBS solution was added to resuspend the cells, and the average fluorescence intensity (MFI) of PE channel was detected by ZE5 flow cytometry (Bio-Rad, ZE5).

Data processing:

The $EC_{50}$ of the affinity of each candidate antibody for tumor cells was calculated by the GraphPad PRISM 8.0 log(agonist) vs. response-viable slope (four parameters) formula taking the logarithmic value of the mutant antibody concentration as the X-axis coordinate and the MFI as the ordinate, as shown in Table 5 below:

Table 5. Affinity of HU6DL mutants for NCI-N87, HCC827, SW780 and RT4 tumor cells ($EC_{50}$)

| Name | NCI-N87 | | HCC827 | | SW780 | | RT4 | |
|---|---|---|---|---|---|---|---|---|
| | TOP MFI | $EC_{50}$ (nM) | TOP MFI | $EC_{50}$ (nM) | TOP MFI | $EC_{50}$ (nM) | TOP MFI | $EC_{50}$ (nM) |
| HU6DL.R54 | 2693 | 1.12 | 6088 | 1.36 | 6581 | 1.22 | 3231 | 1.21 |
| HU6DL.T54 | 1703 | 2.25 | 4563 | 1.46 | 4850 | 1.20 | 2254 | 1.31 |

Experimental conclusion:

The above data showed that the HU6DL mutants of the present invention had good affinity for the NCI-N87, HCC827, SW780 and RT4 tumor cells.

**Example 4: Experiment on HU6DL mutant-mediated TROP2 endocytosis**

Experiment purpose:

Flow cytometry was used to evaluate the antibody endocytic activity of HU6DL mutants on tumor cell lines expressing Trop-2 antigen.

Experimental steps:

Accutase (*Sigma, cat: A6964*) digestion solution was used to treat gastric cancer cell NCI-N87 (purchased from the Cell Bank of the Chinese Academy of Sciences, TCHu130) in good growth status, a single cell suspension was prepared with 2% FBS (diluted in DBPS, pH 7.4) solution, and the cell density was adjusted to 1 $\times$ $10^7$ cells/mL. The suspension was evenly distributed to 96-well V-shaped bottom plate at 100 $\mu$L/ well and a solution of candidate antibody was added at final concentration of 20 $\mu$g/mL, and the mixture was mixed evenly and incubated at 4°C for 1 h,

and centrifuged at 4°C at 300 g $\times$ 5 min. A solution of mouse anti-human IgG Fc secondary antibody labeled with PE (*Biolegend, cat: 409304*) diluted at the ratio of 5 $\mu$L/$10^6$ cells was added at 100 $\mu$L/well, and the mixture was incubated at 4°C for 1 h,

centrifuged at 4°C at 300 g × 5 min, and washed twice. 1 mL of pre-heated complete culture medium was used to resuspend the cell pellet, and the obtained suspension was divided into four equal parts, which were set as 0 min group, blank group, 30 min group and 120 min group respectively. The 0 min group and the blank group were taken and put on ice. The rest were placed in a 37°C incubator for endocytosis for 30 min and 120 min respectively. The corresponding groups were taken out at the corresponding time points and placed on ice to pre-cool for 5 min. All treatment groups were centrifuged and the supernatant (4°C, 1500 rpm × 5 min) was discarded, FACS buffer was used for washing once, and the supernatant was discarded. 250 μL of strip buffer was added to all treatment groups except for the 0 min group, the mixture was incubated at room temperature for 8 min and centrifuged (4°C, 1500 rpm × 5 min), and the supernatant was discarded. FACS buffer was used for washing twice, and the supernatant was removed. 80 μL of 2% FBS was added to each group of samples to resuspend the cells, and the fluorescence signals of the samples were detected by ZE5 flow cytometry (Bio-Rad, ZE5).

Data processing:
the endocytosis efficiency of each candidate antibody was calculated according to this formula: antibody endocytosis percentage (%) = (MIF of treatment group - MFI of blank group)/(MFI of 0 min group - MFI of blank group) * 100%. The endocytosis rate of the HU6DL mutant detected using the above method was shown in Table 6 below:

Table 6. Endocytosis efficiency of HU6DL mutants on NCI-N87 cells

| Name | Antibody endocytosis rate (%) | |
|---|---|---|
| | 30 min | 120 min |
| HU6DL.R54 | 64 | 62 |
| HU6DL.Y54 | 71 | 71 |
| HU6DL.Q54 | 71 | 71 |
| HU6DL.L54 | 76 | 72 |
| HU6DL.T54 | 63 | 69 |
| HU6DL.I54 | 65 | 66 |
| HU6DL.F54 | 72 | 75 |
| HU6DL.E54 | 75 | 72 |
| HU6DL.A54 | 72 | 70 |

Experimental conclusion:

The above data showed that the HU6DL mutants of the present invention mediated the good endocytosis of TROP-2 protein in gastric cancer cell NCI-N87.

**Example 5: Detection of impurity components in HU6DL mutants**

Experiment purpose: Capillary electrophoresis was used to detect and compare the content and change levels of impurity peaks in the mutated antibodies based on the Maurice-nrCE-SDS method.

Experimental steps

1. Sample preparation:

(1) Liquid change and concentration for sample: If the protein concentration of the sample was less than 5 mg/ml, or the salt concentration of the buffer for the sample was high, liquid change and concentration should be performed for the sample to ensure that the protein concentration was around 5 mg/ml, and the salt concentration in the sample should be less than 50 mM.

(2) Non-reducing CE sample processing: A sample was added to a EP tube. The amount of protein for each sample was 50 μg. 1 μl of 10 kD internal standard (Protein Simple, 046-144) was added, 2.5 μl of 250 mM IAM (Sigma, I1149-5G) was added, and 1 × sample buffer (Protein Simple, 046-567) was added to a final volume of 50 μl. (3) After shaking and mixing evenly, the mixture was heated at 70°C, incubated for 10 min, then take out, incubated on ice for 5 min and cooled, and then centrifuged at 12000 rpm for 5 min. After centrifugation, 35 μl of the supernatant was transferred to a 96-well sample plate that matches the instrument, and then centrifuged at 1000 rpm for 5 min. The 96-well sample plate was placed in Maurice (Protein Simple) to prepare for sample loading and analysis.

2. On-machine detection

The instrument was turned on and the software was opened, and the instrument operation steps were followed to perform instrument self-test. The capillary cartridge was installed, and corresponding reagents were prepared and put into the corresponding positions of the instrument. According to the instrument operation steps, the corresponding parameters were set to perform non-reducing CE analysis. Sample sequence setting was performed, the corresponding sequence was edited according to the sample name, and the number of samples for each sequence did not exceed 48. After the sequence editing was completed, "start" was clicked to start sequence detection.

The following formula was used to calculate the content of the main peak of the sample and the content of the impurity peak of the sample.

$$\text{Non-reducing purity (\%)} = \frac{\text{CA main peak}}{\text{CA total}} \times 100\%$$

Notes: In the formula, the non-reducing purity was the corrected peak area percentage of the main peak; $CA_{main\ peak}$ was the corrected peak area of the main peak; $CA_{total}$ was the sum of the corrected peak areas of the main peak and the impurity peak.

Table 7. Contents of main peak and impurity peak of HU6DL mutant

| Sample | Main peak content (%) | Impurity peak content (%) |
|---|---|---|
| HU6DL T54 | 93.62 | NA |

The experimental results showed that the main peak content of sample HU6DL T54 reached 93.62%, no impurities were detected, and the sample had very good purity.

**Example 6 Preparation of compound 1**

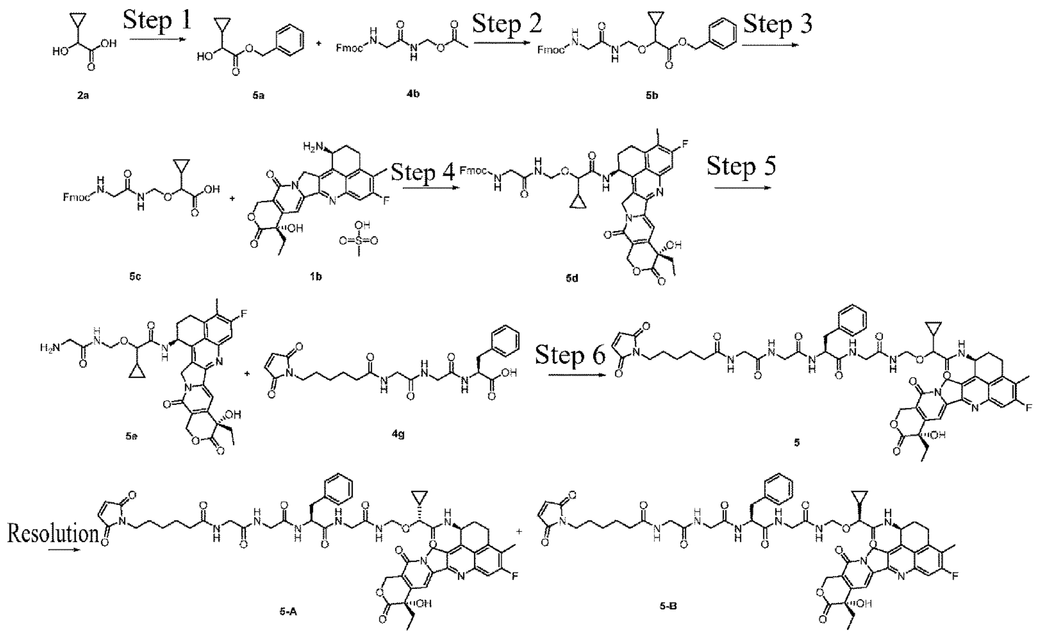


Step 1: **2a** (2 g, 17.2 mmol) was dissolved in 75 mL of acetonitrile, and potassium carbonate (9.27 g, 67.2 mmol), benzyl bromide (20 mL, 167.2 mmol) and tetrabutylammonium iodide (620 mg, 1.68 mmol) were added in sequence. The reaction liquid was stirred at room temperature for 48 h, filtered through diatomaceous earth, and the filter cake was rinsed with ethyl acetate (20 ml). The combined filtrate was concentrated under reduced pressure, and the obtained residue was purified by silica gel column chromatography using developer system C to obtain the product **5a** (3.2 g, yield: 90.1%).

Step 2: **5a** (181.3 mg, 0.879 mmol) and **4b** (270 mg, 0.733 mmol) were added to a reaction flask, 6 mL of tetrahydrofuran was added, argon replacement was performed three times, and the mixture was cooled to 0-5°C under ice-water bath; potassium tert-butoxide (164 mg, 1.46 mmol) was added, the ice bath was removed, and the mixture was warmed to room temperature and stirred for 40 min; 15 mL of ice water was added, the mixture was extracted with ethyl acetate (40 mL × 2) and chloroform (20 mL × 5), and the organic phases were combined and concentrated. The obtained residue was dissolved in 6 mL of dioxane, 3 mL of water was added, sodium bicarbonate (73.8 mg, 0.879 mmol) and 9-fluorenylmethyl chloroformate (190 mg, 0.734 mmol) were added, and the mixture was stirred at room temperature for 2 h. 30 mL of water was added, the mixture was extracted with ethyl acetate (20 mL × 3), the organic phase was washed with saturated sodium chloride solution (30 mL), dried over anhydrous sodium sulfate and filtered, and the filtrate was concentrated under reduced pressure. The obtained residue was purified by silica gel column chromatography using developer system C to obtain the product **5b** benzyl 10-cyclopropyl-1-(9H fluoren-9-yl)-3,6-dioxo-2,9-dioxa-4,7-diazaundecano-11-ate (73 mg, yield: 19.4%).

MS m/z (ESI): 515.0 [M+1].

Step 3: **5b** (30 mg, 0.058 mmol) was dissolved in 6.75 mL of a mixed solvent of tetrahydrofuran and ethyl acetate (V : V = 2 : 1), and palladium on carbon (18 mg, content 10%, dry type) was added, hydrogen replacement was performed three times, and the mixture was reacted under strirring at room temperature for 1 h. The reaction liquid was filtered through diatomaceous earth, the filter cake was rinsed with ethyl acetate, and the filtrate was concentrated to obtain the crude product **5c** 10-cyclopropyl-1-(9*H*-fluorene-9-yl)-3,6-dioxo-2,9-dioxa-4,7-diazaundecan-11-oic acid (20 mg), and the product was directly used in the next reaction without purification.

MS m/z (ESI): 424.9 [M+1].

Step 4: **1b** (15 mg, 28.2 μmol) was added to a reaction flask, 1.5 mL of *N,N*-dimethylformamide was added, argon replacement was performed three times, then the mixture was cooled to 0-5°C under ice-water bath; a drop of triethylamine was add dropwise, the crude product **5c** (20 mg, 47.1 μmol) was added, 4-(4,6-dimethoxy-1,3,5-triazin-2-yl)-4-methyl morpholinium chloride (25.4 mg, 86.2 μmol) was added, and the mixture was reacted under strirring under ice bath for 40 min. 15 mL of water was added, the mixture was extracted with ethyl acetate (20 mL × 3), and the organic phases were combined. The organic phase was washed with saturated sodium chloride solution (20 mL × 2), dried over anhydrous sodium sulfate and filtered, and the filtrate was concentrated under reduced pressure. The obtained residue was purified by thin layer chromatography using developer system B to obtain the title product **5d** (9*H* fluoren-9-yl)methyl (2-(((1-cyclopropyl-2-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-2-oxoethoxy)methyl)amino)-2-oxoethyl)carbamate

(23.7 mg, yield: 78.9%).

MS m/z (ESI): 842.1[M+1].

Step 5: **5d** (30 mg, 35.7 μmol) was dissolved in 3 mL of dichloromethane, 1.5 mL of diethylamine was added, and the mixture was stirred at room temperature for 2 h. The reaction liquid was concentrated under reduced pressure, and 1.5 mL of toluene was added and the mixture was concentrated under reduced pressure, which was repeated twice. 4.5 mL of n-hexane was added to the residue to make a slurry. After standing, the supernatant was poured out and the solid was retained. The solid residue was concentrated under reduced pressure and dried with an oil pump to obtain the crude product **5e** 2-((2-aminoacetamido)methoxy)-2-cyclopropyl-*N*-((1*S*,9*S*)-9-ethyl-5-fluoro-9-hydroxy-4-methyl-10,13-dioxo-2,3,9,10,13,15-hexahydro-1*H*,12*H*-benzo[de]pyrano[3',4':6,7]indolizino[1,2-b]quinolin-1-yl)acetamide (23 mg), and the product was used directly in the next reaction without purification.

MS m/z (ESI): 638.0[M+18].

Step 6: the crude product **5e** (20 mg, 32.3 μmol) was dissolved in 1 mL of *N,N*-dimethylformamide, argon replacement was performed three times, then the mixture was cooled to 0-5°C under ice-water bath; a solution of **4g** (31.8 mg, 67.3 μmol) in 0.5 mL of N,N-dimethylformamide was added, 4-(4,6-dimethoxy-1,3,5-triazin-2-yl)-4-methyl morpholinium chloride (27.8 mg, 94.3 μmol) was added and the mixture was reacted under stirring under ice bath for 10 min; the ice bath was removed, and the reaction liquid was warmed to room temperature and stirred for 1 h, the reaction generated compound **5.** The reaction liquid was purified by high performance liquid chromatography (separation conditions: chromatographic column: XBridge Prep C18 OBD 5 um 19*250 mm; mobile phase: A-Water (10 mmol $NH_4OAc$): B-acetonitrile, gradient elution, flow rate: 18 mL/min), the corresponding components were collected and concentrated under reduced pressure to obtain products 5-A and 5-B (3.6 mg, 2.6 mg).

MS m/z (ESI): 1074.4 [M+1].

Single configuration compound 5-A (shorter retention time):
UPLC analysis: retention time 1.14 min, purity: 85% (chromatographic column: ACQUITY UPLC BEHC18 1.7 um 2.1*50 mm, mobile phase: A-water (5 mmol NHaOAc), B-acetonitrile).

$^1H$ NMR (400 MHz, DMSO-$d_6$): δ 8.60 (t, 1H), 8.51-8.49 (d, 1H), 8.32-8.24 (m, 1H), 8.13-8.02 (m, 2H), 8.02-7.96 (m, 1H), 7.82-7.75 (m, 1H), 7.31 (s, 1H), 7.26-7.15 (m, 4H), 6.99 (s, 1H), 6.55-6.48 (m, 1H), 5.65-5.54 (m, 1H), 5.41 (s, 2H), 5.35-5.15 (m, 3H), 4.74-4.62 (m, 2H), 4.54-4.40 (m, 2H), 3.76-3.64 (m, 4H), 3.62-3.48 (m, 2H), 3.20-3.07 (m, 2H), 3.04-2.94 (m, 2H), 2.80-2.62 (m, 2H), 2.45-2.30 (m, 3H), 2.25-2.15 (m, 2H), 2.15-2.04 (m, 2H), 1.93-1.78 (m, 2H), 1.52-1.39 (m, 3H), 1.34-1.12 (m, 5H), 0.87 (t, 3H), 0.64-0.38 (m, 4H).

Single configuration compound 5-B (longer retention time):
UPLC analysis: retention time 1.16 min, purity: 89% (chromatographic column: ACQUITY UPLC BEHC18 1.7 um 2.1*50 mm, mobile phase: A-water (5 mmol NHaOAc), B-acetonitrile).

$^1H$ NMR (400 MHz, DMSO-$d_6$): δ 8.68-8.60 (m, 1H), 8.58-8.50 (m, 1H), 8.32-8.24 (m, 1H), 8.13-8.02 (m, 2H), 8.02-7.94 (m, 1H), 7.82-7.75 (m, 1H), 7.31 (s, 1H), 7.26-7.13 (m, 4H), 6.99 (s, 1H), 6.55-6.48 (m, 1H), 5.60-5.50 (m, 1H), 5.41 (s, 2H), 5.35-5.15 (m, 3H), 4.78-4.68 (m, 1H), 4.60-4.40 (m, 2H), 3.76-3.58 (m, 4H), 3.58-3.48 (m, 1H), 3.20-3.10 (m, 2H), 3.08-2.97 (m, 2H), 2.80-2.72 (m, 2H), 2.45-2.30 (m, 3H), 2.25-2.13 (m, 2H), 2.13-2.04 (m, 2H), 2.03-1.94 (m, 2H), 1.91-1.78 (m, 2H), 1.52-1.39 (m, 3H), 1.34-1.12 (m, 5H), 0.91-0.79 (m, 3H), 0.53-0.34 (m, 4H).

Referring to Intermediate 5 for the preparation methods of other intermediates.

At 37°C, the prepared aqueous solution of tris(2-carboxyethyl)phosphine (10 mM, 0.347 mL, 3.47 μmol) was added to a solution of antibody HU6DL.R54 in aqueous PBS buffer (0.05 M aqueous PBS buffer with pH = 6.5; 7.3 ml, 13.8 mg/ml, 0.681 μmol), and the mixture was placed in a shaker under water bath and reacted at 37°C for 3 h under shaken, and then the reaction was stopped. The reaction liquid was cooled to 25°C under water bath, diluted to 14.0 ml, and 3.3 ml of the diluted reaction liquid was take out for reaction.

Compound **5-A** (5.0 mg, 2.75 μmol) was dissolved in 0.15 mL of DMSO, the resulting mixture was added to the above-mentioned 3.3 ml of solution, and the mixture was placed in a shaker under water bath and reacted at 25°C for 3 h under shaken, and then the reaction was stopped. The reaction liquid was desalted and purified by Sephadex G25 gel column (elution phase: 0.05 M aqueous PBS buffer solution with pH 6.5, containing 0.001 M EDTA) to obtain compound 1 in PBS buffer solution (1.45 mg/mL, 17 mL), which was refrigerated and stored at 4°C.

The average value y was determined using the UV method. The cuvette containing the sodium succinate buffer was placed in the reference absorption cell and the sample measurement absorption cell respectively, then the solvent blank was deducted, and the cuvette containing the solution of test article was placed in the sample measurement absorption cell, and the absorbance at 280 nm and 370 nm was determined.

Data processing:

By establishing a standard curve, the absorption at 280 nm wavelength was measured to determine the antibody content Cmab, and measure the absorption at 370 nm wavelength to determine the small molecule content CDrug.

The average drug load y = CDrug/Cmab.

The drug load of the product was determined by the above method, and a sample of compound 1 (y = 4) was obtained through UV-HPLC purification.

Referring to compound 1 for the preparation method of compound 2 to compound 9.

**Example 7 Synthesis of compound 10**

Synthesis of compound a

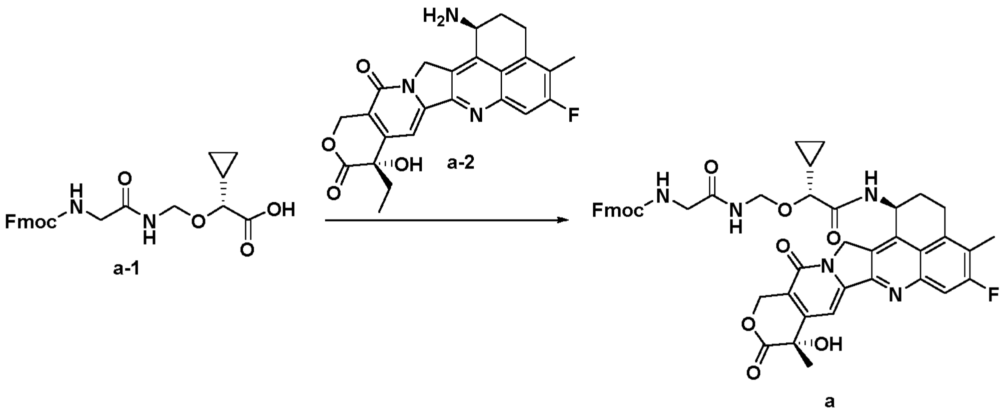

Raw material a-1 (4.1 g, 9.71mmol) and raw material a-2 (4.3 g, 8.09 mmol, containing 4% amino isomer impurities) were taken and placed in a 250 mL reaction bottle. DCM (54 mL) and MeOH (18 mL) were added under nitrogen protection, and the mixture was stirred and cooled to 0°C. DMTMM (3.6 g, 12.1 mmol) and triethylamine (2.5 g, 24.2 mmol) were added, and the mixture was reacted under stirring at 0°C for 1 h. HPLC showed that the reaction of raw material a-2 was complete, and the reaction liquid was evaporated under reduced pressure (< 25°C). MTBE (120 mL) was added and the mixture was stirred and slurried (slurry), and the solution was poured out and filtered. 120 mL of MTBE was added to the slurry again, and the mixture was slurried (solid) and filtered. The filter cake was washed with water (60 mL × 2) and dried in the air to obtain a crude product. The crude product was dissolved in dichloromethane and methanol, and the mixture was separated by wet column chromatography twice (eluent DCM : MeOH = 40 : 1-20 : 1) to obtain the pure compound a (6.2 g, 7.37 mmol), purity: 99.3%, yield 91%.

Synthesis of compound b

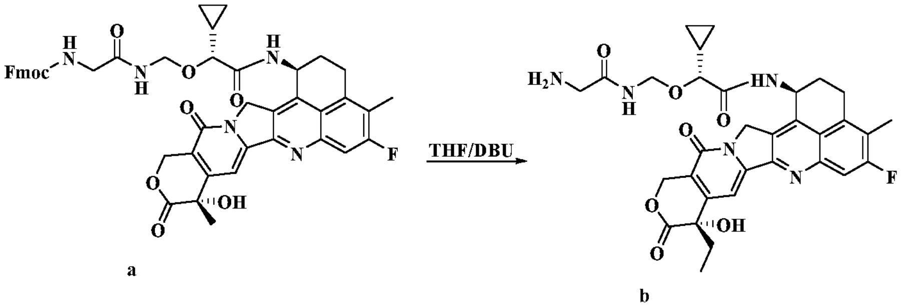


Compound a (5.7 g, 6.77 mmol) was taken and placed in a 500 mL three-necked flask. Dry THF (114 mL) was added under nitrogen protection and dissolved under stirring, and the internal temperature was cooled to about -10°C. DBU (3.09 g, 20.31 mmol) was added, the internal temperature was kept at -10°C to -5°C during the dropwise addition, and the addition was completed in 5 min. After the dropwise addition, the internal temperature was kept at -10°C to -5°C,

and the mixture was reacted for 2.5 h before the solid was precipitated.

The internal temperature was cooled to -20°C, MTBE (114 mL) was added during which the internal temperature was kept at -20°C to -10°C. The product was completely precipitated and filtered, and the filter cake was washed with MTBE (57 mL × 2). After suction filtration until dryness, the crude product compound b (6 g) was obtained, and stored at -78°C for later use.

Synthesis of compound e

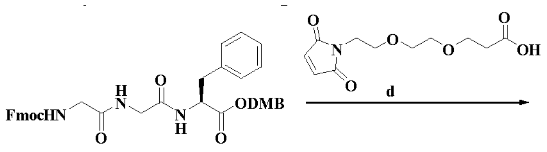

c e

Compound c (651 mg, 1 mmol) was dissolved in 10 mL of DCM, stirring was started while cooling under an ice bath, and DBU (456 mg, 3 mmol) was added dropwise. The reaction was completed after one hour reaction under an ice bath. Compound d (257 mg, 1 mmol) and HATU (420 mg, 1.1 mmol) were added in sequence. After stirring under an ice bath for 30 min, LCMS showed that the reaction was completed. The reaction liquid was concentrated at 25°C, and the residue was purified by reverse-phase column chromatography machine (ACN in $H_2O$, 50% product) to obtain compound e as a reddish-brown solid (130 mg, yield 19%).

MS:691.3 [M+23].

Synthesis of compound f

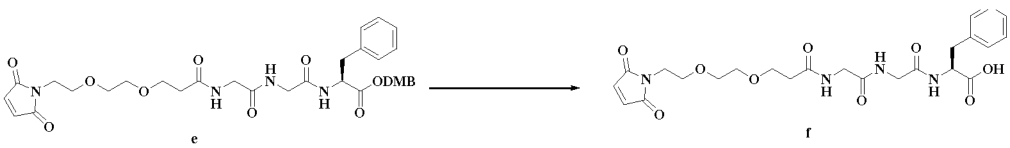
e f

Compound e (130 mg, 0.19 mmol) was dissolved in DCM, anisole (62 mg, 0.57 mmol) and dichloroacetic acid (245 mg, 1.9 mmol) were added, the reaction was stirred at room temperature overnight for a total of 16 h, and sampling was performed for LC-MS control, indicating that the raw materials were completely consumed. The reaction was stopped, the reaction liquid was concentrated at 25°C, and the residue was purified by reverse-phase column chromatography ($ACN/H_2O$, 30% product) to obtain compound f as a pink solid (53 mg, yield 54%).

MS:519.2 [M+1].

Synthesis of compound D

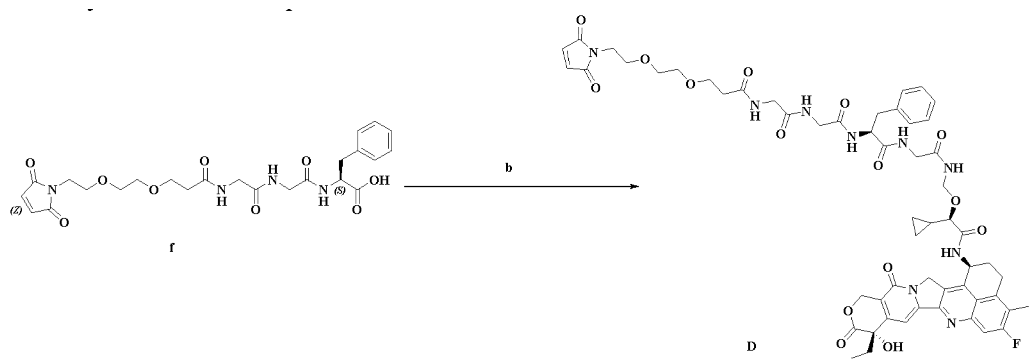

f D

Compound f (23 mg, 0.044 mmol) and compound b (27 mg, 0.044 mmol) were dissolved in DCM (3 mL) and MeOH (1 mL), and the mixture was cooled to -30°C under nitrogen protection. DMTMM (20 mg, 0.067 mmol) was added, and the reaction was carried out at a temperature of -20°C to -10°C for 1 h. Sampling was performed for LC-MS control, indicating that the raw materials were completely consumed. The temperature was controlled at -10°C, 10 mL of water was added to quench the reaction, and 30 mL of DCM was added for layering. The aqueous phase was extracted with DCM/MeOH = 10/1 (50 mL). The organic phases were combined, dried over anhydrous sodium sulfate, and concentrated under reduced pressure at 25°C. The residue was purified by preparative method (ACN/$H_2O$/0.05% FA) to obtain compound D as a white solid (5.8 mg, yield 12%, HPLC purity 98.87%).

MS: 1120.3 [M+1].

The hydrogen spectrum was shown in Fig. 1.

At 37°C, the prepared aqueous solution of tris(2-carboxyethyl)phosphine (10 mM, 0.239 mL, 1.70 μmol) was added to a solution of antibody HU6DL.R54 in aqueous PBS buffer (0.05 M aqueous PBS buffer with pH = 6.5; 7.3 ml, 13.8 mg/ml, 0.681 μmol), and the mixture was placed in a shaker under water bath and reacted at 37°C for 3 h under shaken, and then the reaction was stopped. The reaction liquid was cooled to 25°C under water bath, diluted to 14.0 ml, and 3.3 ml of the diluted reaction liquid was take out for reaction.

Compound **D** (3.0 mg, 3.72 μmol) was dissolved in 0.15 mL of DMSO, the resulting mixture was added to the above-mentioned 3.3 ml of solution, and the mixture was placed in a shaker under water bath and reacted at 25°C for 3 h under shaken, and then the reaction was stopped. The reaction liquid was desalted and purified by Sephadex G25 gel column (elution phase: 0.05 M aqueous PBS buffer solution with pH 6.5, containing 0.001 M EDTA) to obtain compound 10 of the mAb2 antibody conjugate in PBS buffer solution of (1.35 mg/mL, 13 mL), which was refrigerated and stored at 4°C.

The average value y was determined using the UV method. The cuvette containing the sodium succinate buffer was placed in the reference absorption cell and the sample measurement absorption cell respectively, then the solvent blank was deducted, and the cuvette containing the solution of test article was placed in the sample measurement absorption cell, and the absorbance at 280 nm and 370 nm was determined.

Data processing:

By establishing a standard curve, the absorption at 280 nm wavelength was measured to determine the antibody content Cmab, and measure the absorption at 370 nm wavelength to determine the small molecule content CDrug.

$$\text{The average drug load y} = \text{CDrug/Cmab}.$$

The drug load of the product was determined by the above method, and a sample of compound 10 (y = 4) was obtained through UV-HPLC purification.

Referring to compound 10 for the preparation method of compound 11 to compound 18.

**Example 8 Cell killing activity of antibody-drug conjugate**

To evaluate the killing effect of the antibody-drug conjugate of the present disclosure on tumor cells, Trop-2 positive cell line MDA-MB-468 was used for evaluation. The cultured MDA-MB-468 monolayer cells was digested with trypsin, culture medium was added to resuspend the digested cells, the cells were counted after centrifugation, the cell density was adjusted to $4 \times 10^4$ cells/mL with complete culture medium, and then the cells were plated in 60 wells in the middle of a white 96-well plate with transparent bottom (Corning, cat: 3610) at 50 μl/well, and the number of cells was 2000 cells/well. Culture medium was added to the edge wells at 100 μl/well. The cell plate was placed in an incubator at 37°C and 5% $CO_2$ for culture overnight. The next day, complete culture medium was used to dilute the antibody-drug conjugate working solution in a 96-well V-bottom plate (Corning, cat: 3894). The starting concentration was 10000 nM, 10-fold dilution was performed for 9 concentration gradients. After preparation was completed, the solution was added to a white 96-well plate at 50 μl/well in duplicate. The cell plate was placed in an incubator at 37°C and 5% $CO_2$ for further culture for 6 days. On the 6th day of the experiment, reading was performed: the cell culture plate was taken out, and balanced to room temperature, 50 μl of CellTiter-Glo® cell viability detection reagent (Promega, Cat#: G7573) was added to each well, the mixture was shaken and mixed evenly, the plate was placed in the dark for standing for 20 min, and then the chemiluminescence signal value was detected at 490 nm and 500 ms/well by microplate reader. The inhibition rate (%) was calculated from the luminescence signal value,
the calculation formula was:

Inhibition rate (%) = (1 - signal value of sample well / average signal value of control well) $\times$ 100.

The sample well was a cell well treated with the test drug, and the control well was a cell well treated with complete culture medium.

Curve fitting: According to the inhibition rate (%) corresponding to each concentration, curve fitting was performed to obtain $IC_{50}$ value use the log(inhibitor) vs. response -- Variable slope (four parameters) equation in GraphPad Prism 6.0, with the logarithmic value of concentration as the X-axis and the inhibition rate as the Y-axis. The calculation equation was:

$$Y = Bottom + (Top - Bottom)/(1 + 10^{\wedge}((LogIC_{50}-X)*HillSlope)).$$

The experimental results were as shown in the following table.

Table 8. Killing effect of antibody-drug conjugate on tumor cells

| | **$IC_{50}$ (nM)** |
|---|---|
| **Antibody-drug conjugate (ADC)** | **MDA-MB-468** |
| Compound 14 (y = 4) | 3.4 |
| Compound 5 (y = 4) | 5.7 |
| hIgG1 - Compound 5-A (y = 4) | 164.2 |

By comparing the cell killing $IC_{50}$, the experimental results showed: relative to the negative control, the antibody-conjugated drugs of the present invention all had very strong cell killing effects.

**Example 9 Pharmacokinetics of antibody-drug conjugate**

The BALB/c mouse model was used to evaluate the drug metabolism of anti-Trop-2 antibody-drug conjugate Tro-2 ADC in mice. BALB/c mice with an average weight of 18-22 g and 6-8 weeks of age were randomly divided into 3 groups, with 3 animals in each group. The tested Trop-2 ADC antibody-drug conjugates were all administrated with 4 mpk at single IV, and blood was collected at 0.5, 2, 4, 8, 24, 48, 72, 96, 144, and 240 h respectively, and the plasma was separated, frozen and stored in refrigerator at -20°C. Then a 96-well fully transparent plate (Corning, cat: 3590) with high affinity was coated with anti-ADC polyclonal antibody. Diluted plasma samples to be tested were added, and then the concentration of Trop-2 ADC in mouse plasma was detected by using goat anti-human IgG1 $F(ab')_2$ fragment $F(ab')_2$ secondary antibody labeled with HRP, and the PK parameter was analyzed by using non-compartment model and intravascular administration model in PKSolver software. The experimental results were detailed in the table below

Table 9. Pharmacokinetic parameters of antibody-drug conjugates

| **PK parameters** | **Unit** | Compound 14 (y = 4) | Compound 5 (y = 4) |
|---|---|---|---|
| t1/2 | h | 189.5 | 179.8 |
| Tmax | h | 0.5 | 0.5 |
| Cmax | ng/ml | 411.8 | 333.8 |
| AUC 0-t | ng/ml*h | 49388.7 | 40735.8 |
| MRT 0-inf_obs | h | 265.3 | 259.1 |
| Cl_obs | (mg/kg)/(ng/ml)/h | 8.099E-05 | 9.819E-05 |

Combining the values of parameters such as the half-life t1/2, the time to reach peak blood drug concentration Tmax, the blood drug concentration Cmax, and the clearance rate Cl_obs, the antibody-drug conjugates of the present invention showed good metabolic characteristics.

**Example 10 *In vivo* anti-tumor effect of antibody-drug conjugate**

In order to further study the inhibitory effect of antibody-drug conjugate on tumors formed *in vivo,* the Trop-2 positive tumor cell human gastric cancer NCI-N87 tumor cell transplant mouse model was used to evaluate the inhibitory effect of candidate molecule on tumor proliferation *in vivo.* $5 \times 10^6$ NCI-N87 cells were injected subcutaneously into 8-week-old nude mice (Balb/c Nude) weighing about 18-20 mg. After 10 days, the average tumor volume reached 160 $mm^3$ and grouping was performed, and the antibody-drug conjugate was started to be injected intravenously every 2 weeks at a dose of 4 mg/kg. The control used human IgG1 isotype control antibody at a dose of 4 mg/kg. There were 5 mice in each group of the control group or the administration group. The tumor inhibition rate was calculated by measuring the tumor volume.

Tumor inhibition rate = 100% - (tumor valume of the administration group on day 28 - tumor volume of the administration group on day 0)/(tumor volume of the control group on day 28 - tumor volume of the control group on day 0).

Table 10. Killing effect of antibody-drug conjugate on tumor

| **Administration group** | **Dosage** | **Frequency** | **Tumor inhibition rate (%)** |
|---|---|---|---|
| hIgG1 iso | 4 mg/kg | Q2W | - |
| Compound 14 (y = 4) | 4 mg/kg | Q2W | 122.44 |
| Compound 5 (y = 4) | 4 mg/kg | Q2W | 141.21 |

Experimental results showed that when injected once every 2 weeks at a dose of 4 mg/kg, the antibody-drug conjugates of the present invention all showed good tumor inhibition effect. In addition, the antibody-drug conjugates of the present invention also showed good tumor inhibition rate at low doses.

**Example 11 Dose-dependent anti-tumor effect of antibody-drug conjugate *in vivo***

In order to further study the inhibitory effect of antibody-drug conjugate on tumors formed *in vivo,* the Trop-2 positive tumor cell human gastric cancer NCI-N87 tumor cell transplant mouse model was used to evaluate the inhibitory effect of candidate molecule on tumor proliferation *in vivo.* $5 \times 10^6$ NCI-N87 cells were injected subcutaneously into 6-week-old nude mice (Balb/c Nude) weighing about 18-22 mg. After 8 days, the average tumor volume reached 175 $mm^3$ and grouping was performed, and the antibody-drug conjugate was started to be injected intravenously every 2 weeks at a dose of 2 mg/kg or 1 mg/kg. The control used human IgG1 isotype control antibody at a dose of 2 mg/kg. There were 5 mice in each group of the control group or the administration group. The tumor inhibition rate was calculated by measuring the tumor volume.

Tumor inhibition rate = 100% - (tumor volume of the administration group on day 28 - tumor volume of the administration group on day 0)/(tumor volume of the control group on day 28 - tumor volume of the control group on day 0).

Table 11. Killing effect of antibody-drug conjugate on tumor

| **Administration group** | **Dosage** | **Frequency** | **Tumor inhibition rate (%)** |
|---|---|---|---|
| hIgG1 iso | 2 mg/kg | Q2W | - |
| Compound 14 (y = 4) | 2 mg/kg | Q2W | 124.25 |
| Compound 14 (y = 4) | 1 mg/kg | Q2W | 85.97 |
| DS-1062-compound D (y = 4) | 1 mg/kg | Q2W | 78.2 |

Experimental results showed that when administered once every 2 weeks at a dose of 2 mg/kg or 1 mg/kg for 28 days, the antibody-drug conjugates of the present invention showed good tumor inhibition effect on the tumor-bearing mouse model of human gastric cancer cell NCI-N87 CDX, and showed a dose-dependent effect.

**Example 12 Study on the *in vivo* efficacy model of antibody-drug conjugate on head and neck cancer squamous cell carcinoma FaDu**

In order to further study the inhibitory effect of antibody-drug conjugate on tumors formed *in vivo,* the Trop-2 positive tumor cell human head and neck cancer squamous cell carcinoma FaDu tumor cell transplant mouse model was used to evaluate the inhibitory effect of candidate molecule on tumor proliferation *in vivo.* The cell suspension mixed with Matrigel in equal proportions was added, and $5 \times 10^6$ FaDu cells per animal were injected subcutaneously into 6-week-old nude mice (Balb/c Nude) weighing about 18-22 mg. After 8 days, the average tumor volume reached 133 $mm^3$ and grouping was performed, and the antibody-drug conjugate was started to be injected intravenously at a single dose of 3 mg/kg or 1 mg/kg. The control used human IgG1 isotype control antibody at a dose of 3 mg/kg. There were 5 mice in each group of the control group or the administration group. The tumor inhibition rate was calculated by measuring the tumor volume.

Tumor inhibition rate = 100% - (tumor volume of the administration group on day 17 - tumor volume of the administration group on day 0)/(tumor volume of the control group on day 17 - tumor volume of the control group on day 0).

Table 12. Killing effect of antibody-drug conjugate on tumor

| Administration group | Dosage | Frequency | Tumor inhibition rate (%) |
|---|---|---|---|
| hIgG1 iso | 3 mg/kg | Single dose | - |
| Compound 14 | 3 mg/kg | Single dose | 200 |
| DS-1062 (DAR4 70%) | 3 mg/kg | Single dose | 200 |
| Compound 14 | 1 mg/kg | Single dose | 200 |
| DS-1062 | 1 mg/kg | Single dose | 74.8 |

The experimental results showed that after random grouping and administration of a single dose, both doses of 3 mg/kg and 1 mg/kg had very obvious tumor inhibition effect on tumor-bearing mouse model of human head and neck cancer squamous cell carcinoma FaDu CDX with a tumor inhibition rate reached 200% 17 days after administration at a dose of 3 mg/kg or 1 mg/kg.

**Example 13 Study on the *in vivo* efficacy model of antibody-drug conjugate on lung cancer Calu-3**

In order to further study the inhibitory effect of antibody-drug conjugate on tumors formed *in vivo,* the Trop-2 positive tumor cell lung cancer Calu-3 tumor cell transplant mouse model was used to evaluate the inhibitory effect of candidate molecule on tumor proliferation *in vivo.* The cell suspension mixed with Matrigel in equal proportions was added, and $2 \times 10^6$ Calu-3 cells per animal were injected subcutaneously into 6-week-old nude mice (Balb/c Nude) weighing about 18-22 mg. After 8 days, the average tumor volume reached 148 $mm^3$ and grouping was performed, and the antibody-drug conjugate was started to be injected intravenously at a single dose of 10 mg/kg. The control used human IgG1 isotype control antibody at a dose of 10 mg/kg. There were 5 mice in each group of the control group or the administration group. The tumor inhibition rate was calculated by measuring the tumor volume.

Tumor inhibition rate = 100% - (tumor volume of the administration group on day 21 - tumor volume of the administration group on day 0)/(tumor volume of the control group on day 21 - tumor volume of the control group on day 0).

Table 13. Killing effect of antibody-drug conjugate on tumor

| Administration group | Dosage | Frequency | Tumor inhibition rate (%) |
|---|---|---|---|
| hIgG1 iso | 10 mg/kg | Single dose | - |
| Compound 14 | 10 mg/kg | Single dose | 100.35 |
| DS-1062 | 10 mg/kg | Single dose | 85.61 |

The experimental results showed that after random grouping and administration of a single dose, and the antibody-drug conjugates of the present invention showed an obvious tumor inhibition effect on tumor-bearing mouse model of human lung cancer Calu-3 CDX with a tumor inhibition rate reached 100% 21 days after administration at a dose of 10 mg/kg.

## Claims

1. An antibody-drug conjugate of general formula (I) or a pharmaceutically acceptable salt or solvent compound thereof,

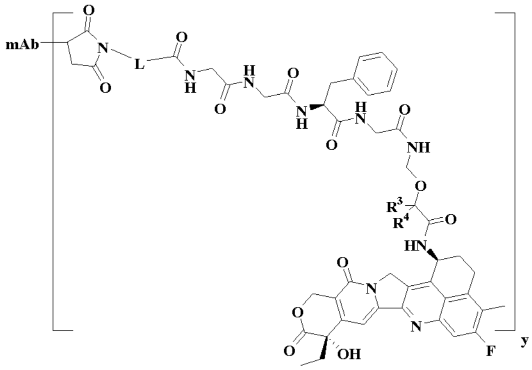

(I)

wherein:

L is $-(CR^1R^2)_m-[X_1-(CR^1R^2)_{n}-X_2]_t(CR^1R^2)_r-$;
$R^1$ or $R^2$ is each independently selected from the group consisting of hydrogen, deuterium, hydroxyl, amino, alkyl, halogen, haloalkyl, deuterated alkyl and hydroxyalkyl, preferably, $R^1$ or $R^2$ is hydrogen;
$X_1$ or $X_2$ is each independently selected from the group consisting of a bond, N, O and S; preferably, $X_1$ or $X_2$ is a bond or O,
m, n, r or t is each independently selected from the group consisting of 1, 2, 3 and 4, preferably, m, n, r or t is each independently 1 or 2;
$R^3$ or $R^4$ is each independently selected from the group consisting of hydrogen, halogen, haloalkyl, deuterated alkyl, cycloalkyl, heterocyclyl, aryl and heteroaryl;
or, $R^3$ and $R^4$ together with the carbon atom to which they are attached form cycloalkyl or heterocyclyl;
y is 1 to 20, preferably 1 to 10, more preferably 2 to 8, and further preferably 4, 6 or 8;
mAb is an anti-TROP-2 antibody or an antigen binding fragment thereof, which comprises:

HCDR1 as shown in SEQ ID NO: 3, HCDR2 as shown in RIDPXDSETHYNQKFKD and HCDR3 as shown in SEQ ID NO: 5; and LCDR1 as shown in SEQ ID NO: 6, LCDR2 as shown in SEQ ID NO: 7 and LCDR3 as shown in SEQ ID NO: 8,
and X is an amino acid residue selected from the group consisting of R, Y, Q, L, T, I, F, E and A.

2. The antibody-drug conjugate or the pharmaceutically acceptable salt or solvent compound thereof according to claim 1, wherein, the TROP-2 antibody or the antigen binding fragments thereof comprises:

HCDR1 as shown in SEQ ID NO: 3, HCDR2 as shown in SEQ ID NO: 9 and HCDR3 as shown in SEQ ID NO: 5; and LCDR1 as shown in SEQ ID NO: 6, LCDR2 as shown in SEQ ID NO: 7 and LCDR3 as shown in SEQ ID NO: 8; or,
HCDR1 as shown in SEQ ID NO: 3, HCDR2 as shown in SEQ ID NO: 10 and HCDR3 as shown in SEQ ID NO: 5; and LCDR1 as shown in SEQ ID NO: 6, LCDR2 as shown in SEQ ID NO: 7 and LCDR3 as shown in SEQ ID NO: 8; or,
HCDR1 as shown in SEQ ID NO: 3, HCDR2 as shown in SEQ ID NO: 11 and HCDR3 as shown in SEQ ID NO: 5; and LCDR1 as shown in SEQ ID NO: 6, LCDR2 as shown in SEQ ID NO: 7 and LCDR3 as shown in SEQ ID NO: 8; or,
HCDR1 as shown in SEQ ID NO: 3, HCDR2 as shown in SEQ ID NO: 12 and HCDR3 as shown in SEQ ID NO:

5; and LCDR1 as shown in SEQ ID NO: 6, LCDR2 as shown in SEQ ID NO: 7 and LCDR3 as shown in SEQ ID NO: 8; or,

HCDR1 as shown in SEQ ID NO: 3, HCDR2 as shown in SEQ ID NO: 13 and HCDR3 as shown in SEQ ID NO: 5; and LCDR1 as shown in SEQ ID NO: 6, LCDR2 as shown in SEQ ID NO: 7 and LCDR3 as shown in SEQ ID NO: 8; or,

HCDR1 as shown in SEQ ID NO: 3, HCDR2 as shown in SEQ ID NO: 14 and HCDR3 as shown in SEQ ID NO: 5; and LCDR1 as shown in SEQ ID NO: 6, LCDR2 as shown in SEQ ID NO: 7 and LCDR3 as shown in SEQ ID NO: 8; or,

HCDR1 as shown in SEQ ID NO: 3, HCDR2 as shown in SEQ ID NO: 15 and HCDR3 as shown in SEQ ID NO: 5; and LCDR1 as shown in SEQ ID NO: 6, LCDR2 as shown in SEQ ID NO: 7 and LCDR3 as shown in SEQ ID NO: 8; or,

HCDR1 as shown in SEQ ID NO: 3, HCDR2 as shown in SEQ ID NO: 16 and HCDR3 as shown in SEQ ID NO: 5; and LCDR1 as shown in SEQ ID NO: 6, LCDR2 as shown in SEQ ID NO: 7 and LCDR3 as shown in SEQ ID NO: 8; or,

HCDR1 as shown in SEQ ID NO: 3, HCDR2 as shown in SEQ ID NO: 17 and HCDR3 as shown in SEQ ID NO: 5; and LCDR1 as shown in SEQ ID NO: 6, LCDR2 as shown in SEQ ID NO: 7 and LCDR3 as shown in SEQ ID NO: 8.

**3.** The antibody-drug conjugate or the pharmaceutically acceptable salt or solvent compound thereof according to claim 1, wherein, the anti-TROP-2 antibody or the antigen binding fragment thereof is selected from the group consisting of a murine antibody or an antigen binding fragment thereof, a chimeric antibody or an antigen binding fragment thereof, a human antibody or an antigen binding fragment thereof, and a humanized antibody or an antigen binding fragment thereof.

**4.** The antibody-drug conjugate or the pharmaceutically acceptable salt or solvent compound thereof according to claim 1, wherein the anti-TROP-2 antibody or the antigen binding fragment thereof further comprises a heavy chain constant region of human IgG1, IgG2, IgG3 or IgG4 or a variant thereof,

preferably, the anti-TROP-2 antibody or the antigen binding fragment thereof further comprises a heavy chain constant region of human IgG1, IgG2 or IgG4;

and more preferably, the anti-TROP-2 antibody or the antigen binding fragment thereof further comprises a heavy chain constant region as shown in SEQ ID NO: 48, or as shown in SEQ ID NO: 1.

**5.** The antibody-drug conjugate or the pharmaceutically acceptable salt or solvent compound thereof according to claim 1, wherein the anti-TROP-2 antibody or the antigen binding fragment thereof further comprises a light chain constant region of a human antibody kappa chain, lambda chain or a variant thereof;

preferably, the anti-TROP-2 antibody or the antigen binding fragment thereof further comprises a light chain constant region of a human antibody kappa chain;

and more preferably, the anti-TROP-2 antibody or the antigen binding fragment thereof further comprises a light chain constant region as shown in SEQ ID NO: 2.

**6.** The antibody-drug conjugate or the pharmaceutically acceptable salt or solvent compound thereof according to any one of claims 1 to 5, wherein, the anti-TROP-2 antibody or the antigen binding fragment thereof comprises a heavy chain variable region selected from the group consisting of the following sequence, or a heavy chain variable region having at least 70%, 75%, 80%, 85%, 90%, 95% or 99% identity to the following sequence: SEQ ID NO: 18, SEQ ID NO: 20, SEQ ID NO: 21, SEQ ID NO: 22, SEQ ID NO: 23, SEQ ID NO: 24, SEQ ID NO: 25, SEQ ID NO: 26 or SEQ ID NO: 27;
and/or, a light chain variable region of or having at least 70%, 75%, 80%, 85%, 90%, 95% or 99% identity to SEQ ID NO: 19.

**7.** The antibody-drug conjugate or the pharmaceutically acceptable salt or solvent compound thereof according to claim 6, wherein, the anti-TROP-2 antibody or the antigen binding fragment thereof comprises:

a heavy chain variable region as shown in SEQ ID NO: 18 and a light chain variable region as shown in SEQ ID NO: 19; or,

a heavy chain variable region as shown in SEQ ID NO: 20 and a light chain variable region as shown in SEQ ID NO: 19; or,

a heavy chain variable region as shown in SEQ ID NO: 21 and a light chain variable region as shown in SEQ ID NO: 19; or,
a heavy chain variable region as shown in SEQ ID NO: 22 and a light chain variable region as shown in SEQ ID NO: 19; or,
a heavy chain variable region as shown in SEQ ID NO: 23 and a light chain variable region as shown in SEQ ID NO: 19; or,
a heavy chain variable region as shown in SEQ ID NO: 24 and a light chain variable region as shown in SEQ ID NO: 19; or,
a heavy chain variable region as shown in SEQ ID NO: 25 and a light chain variable region as shown in SEQ ID NO: 19; or,
a heavy chain variable region as shown in SEQ ID NO: 26 and a light chain variable region as shown in SEQ ID NO: 19; or,
a heavy chain variable region as shown in SEQ ID NO: 27 and a light chain variable region as shown in SEQ ID NO: 19.

**8.** The antibody-drug conjugate or the pharmaceutically acceptable salt or solvent compound thereof according to claim 6, wherein the anti-TROP-2 antibody or the antigen binding fragment thereof comprises a heavy chain selected from the group consisting of the following sequence, or a heavy chain having at least 80%, 85%, 90%, 95% or 99% identity to the following sequence: SEQ ID NO: 28, SEQ ID NO: 30, SEQ ID NO: 31, SEQ ID NO: 32, SEQ ID NO: 33, SEQ ID NO: 34, SEQ ID NO: 35, SEQ ID NO: 36 or SEQ ID NO: 37; and/or, a light chain as shown in or having at least 80%, 85%, 90%, 95% or 99% identity to SEQ ID NO: 29.

**9.** The antibody-drug conjugate or the pharmaceutically acceptable salt or solvent compound thereof according to claim 8, wherein the anti-TROP-2 antibody comprises:

a heavy chain as shown in SEQ ID NO: 28 and a light chain as shown in SEQ ID NO: 29; or,
a heavy chain as shown in SEQ ID NO: 30 and a light chain as shown in SEQ ID NO: 29; or,
a heavy chain as shown in SEQ ID NO: 31 and a light chain as shown in SEQ ID NO: 29; or,
a heavy chain as shown in SEQ ID NO: 32 and a light chain as shown in SEQ ID NO: 29; or,
a heavy chain as shown in SEQ ID NO: 33 and a light chain as shown in SEQ ID NO: 29; or,
a heavy chain as shown in SEQ ID NO: 34 and a light chain as shown in SEQ ID NO: 29; or,
a heavy chain as shown in SEQ ID NO: 35 and a light chain as shown in SEQ ID NO: 29; or,
a heavy chain as shown in SEQ ID NO: 36 and a light chain as shown in SEQ ID NO: 29; or,
a heavy chain as shown in SEQ ID NO: 37 and a light chain as shown in SEQ ID NO: 29.

**10.** The antibody-drug conjugate or the pharmaceutically acceptable salt or solvent compound thereof according to any one of claims 1 to 9, wherein the antibody-drug conjugate or the pharmaceutically acceptable salt or solvent compound thereof is selected from the group consisting of an antibody-drug conjugate of general formula (II) or a pharmaceutically acceptable salt, solvent compound, tautomer, mesomer, racemate, enantiomer, or diastereomer thereof, and a mixture thereof:

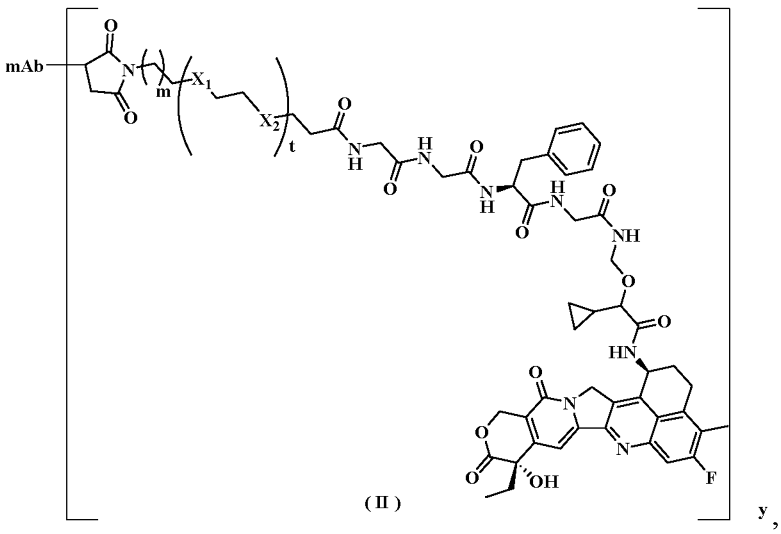

(II)

$X_1$ or $X_2$ is a bond or O;
m is 0 or 1; and
t is 1 or 2.

**11.** The antibody-drug conjugate or the pharmaceutically acceptable salt or solvent compound thereof according to claim 10, wherein the antibody-drug conjugate or the pharmaceutically acceptable salt or solvent compound thereof is selected from the group consisting of an antibody-drug conjugate of general formula (III) or a pharmaceutically acceptable salt or solvent compound thereof:

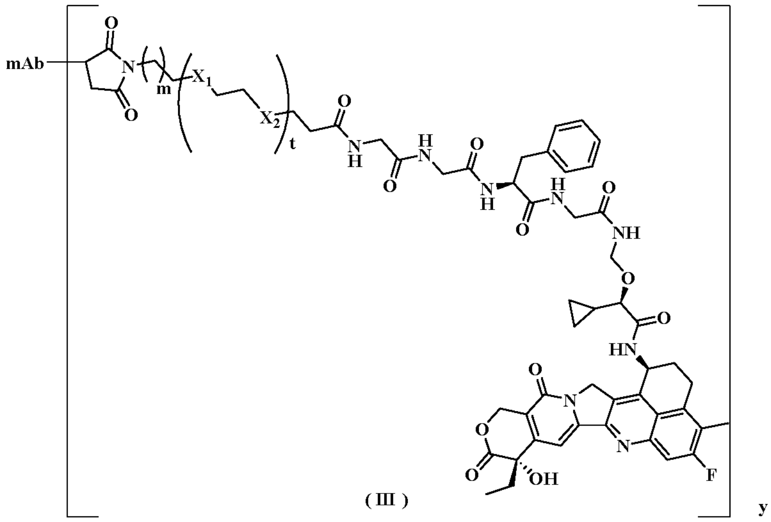

(III)

wherein y is 1 to 10, and preferably 4, 6, 8 or 10.

**12.** The antibody-drug conjugate or the pharmaceutically acceptable salt or solvent compound thereof according to claim 11, wherein the antibody-drug conjugate or the pharmaceutically acceptable salt or solvent compound thereof is selected from the group consisting of the following structures:

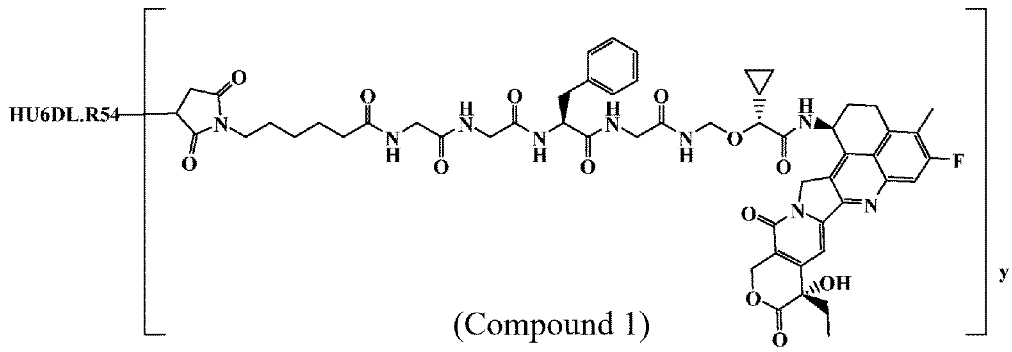

(Compound 1)

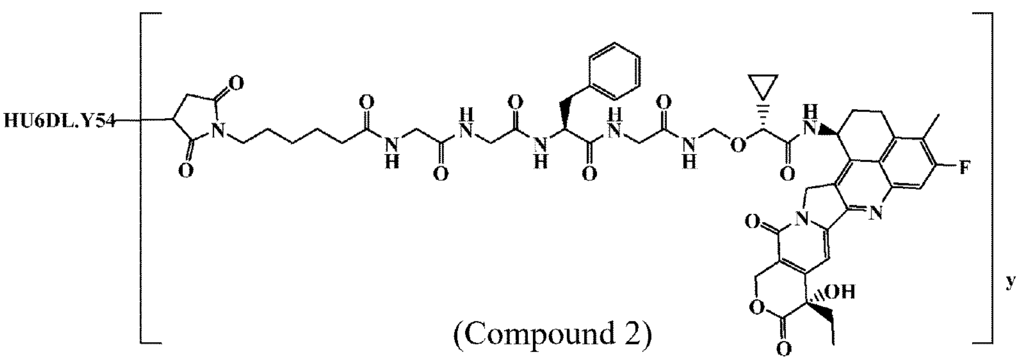

(Compound 2)

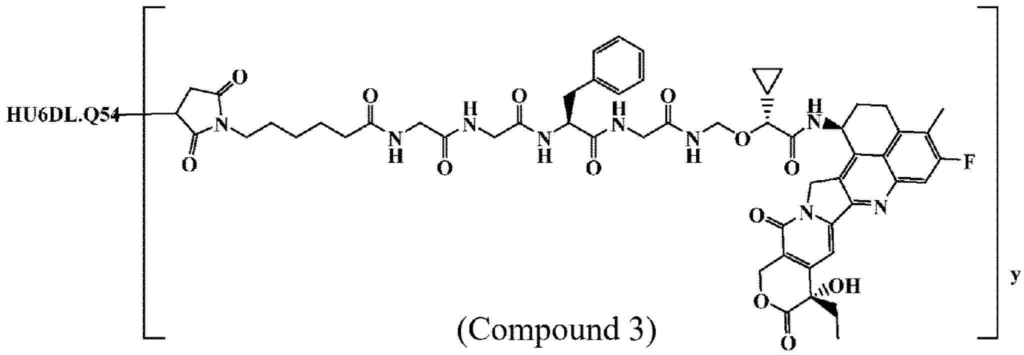

(Compound 3)

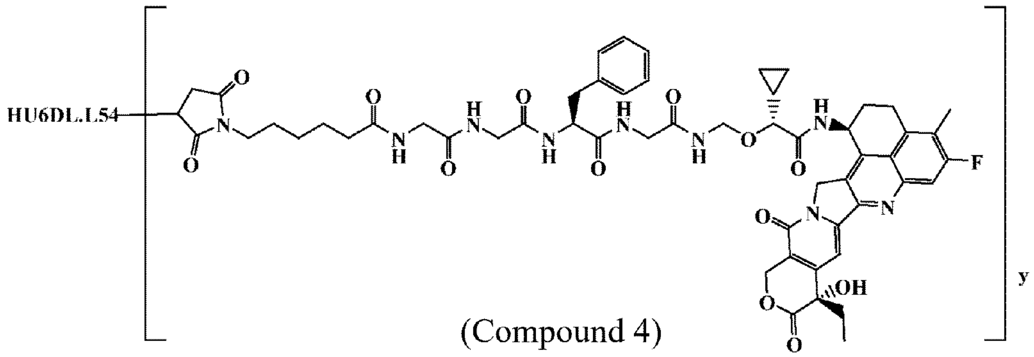

(Compound 4)

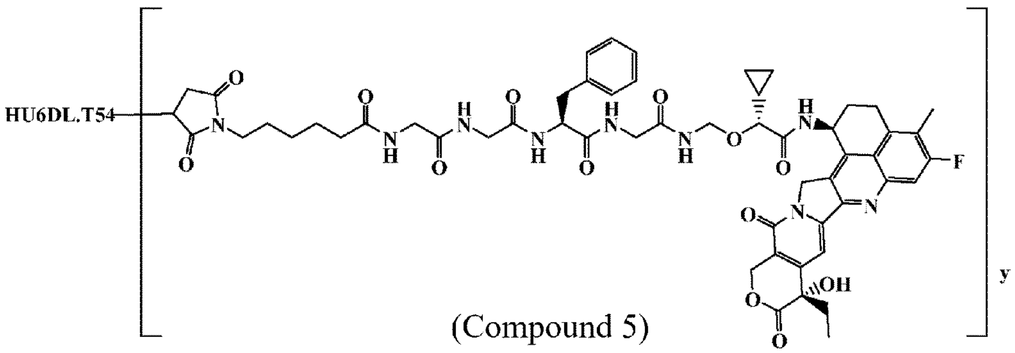

(Compound 5)

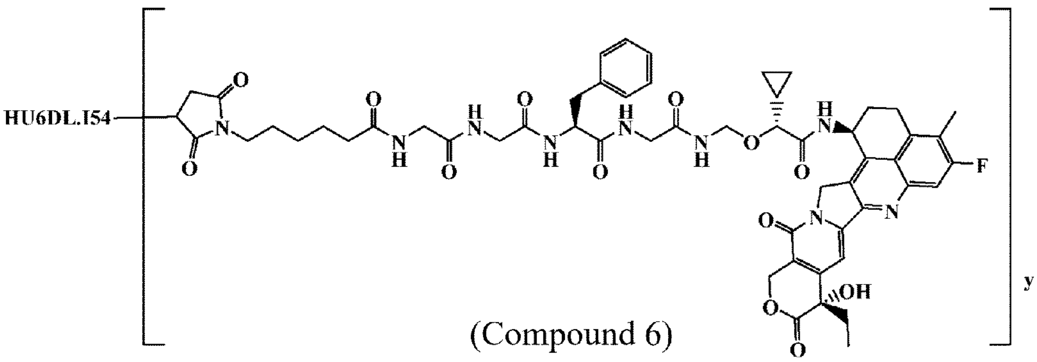

(Compound 6)

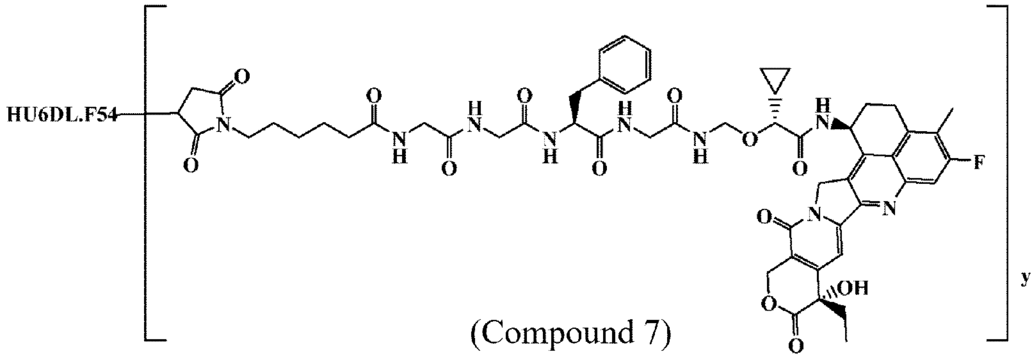

(Compound 7)

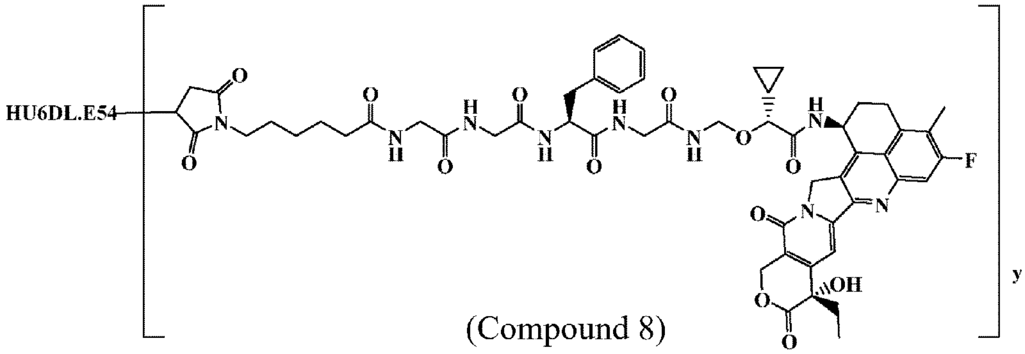

(Compound 8)

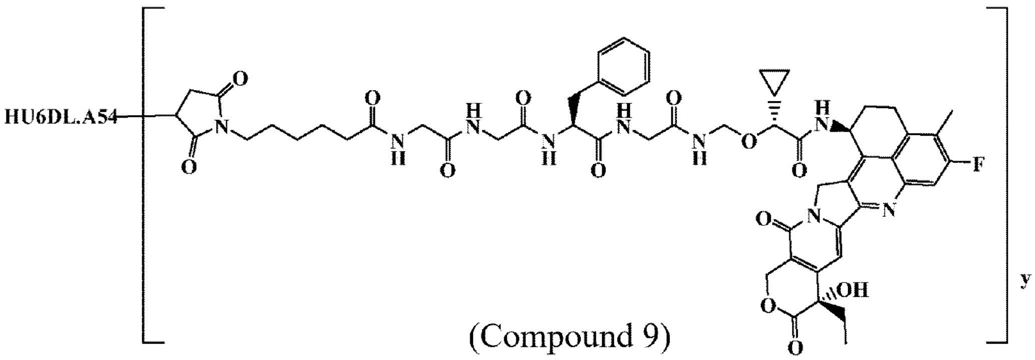

(Compound 9)

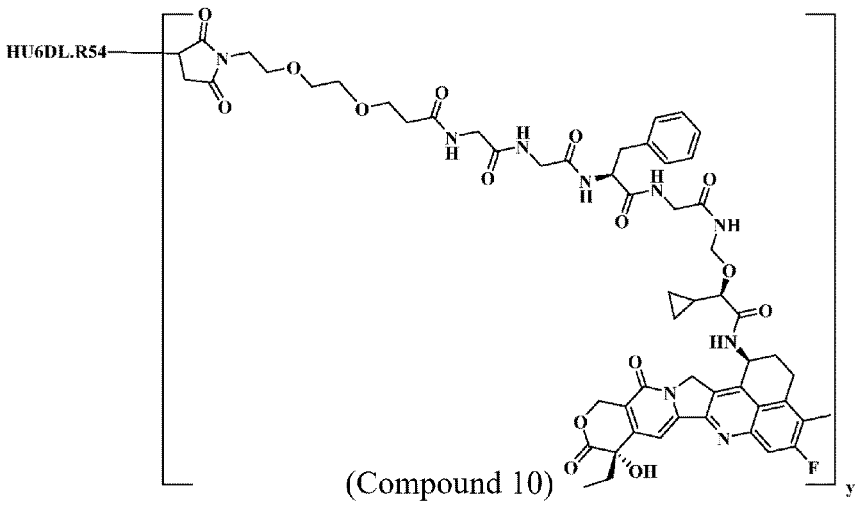

(Compound 10)

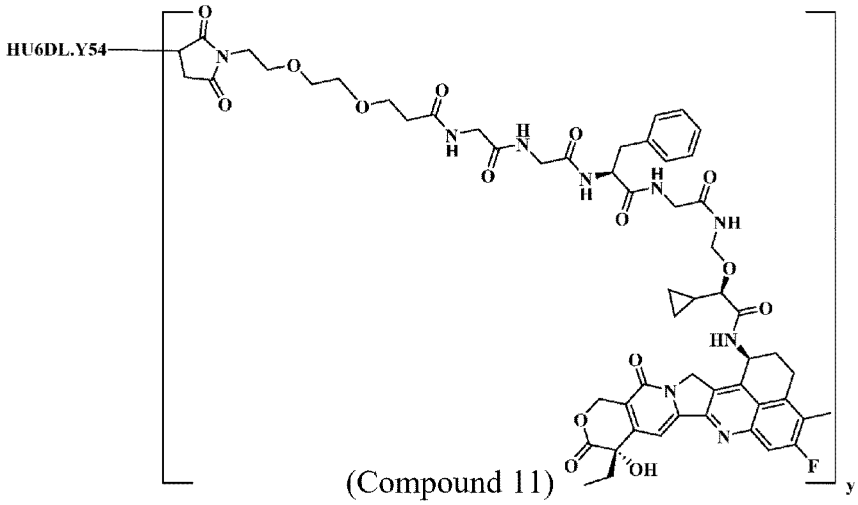

(Compound 11)

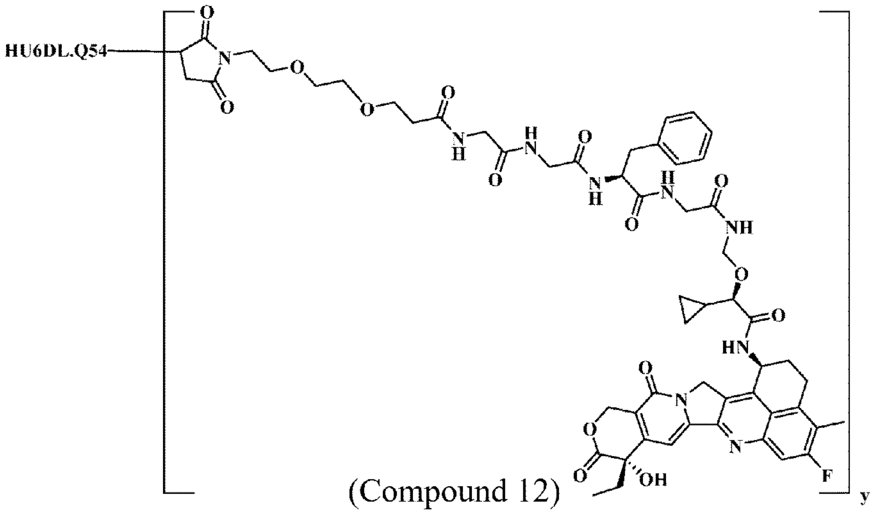

(Compound 12)

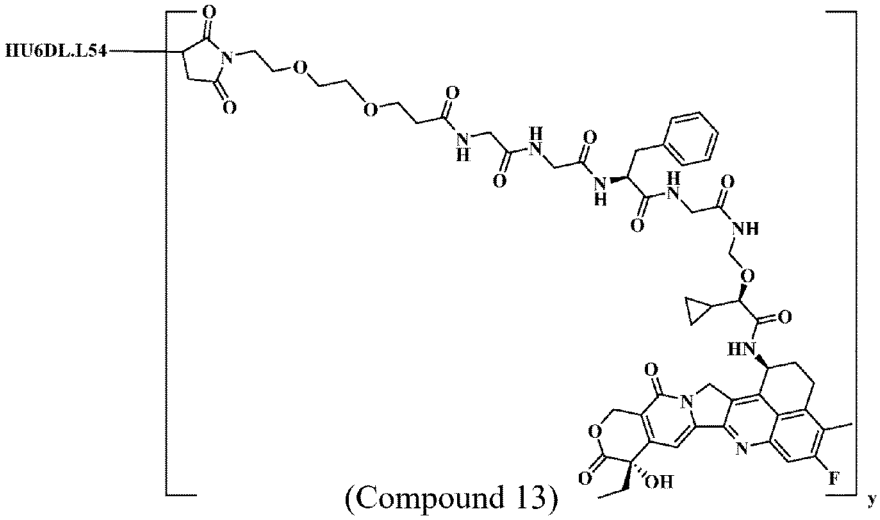

(Compound 13)

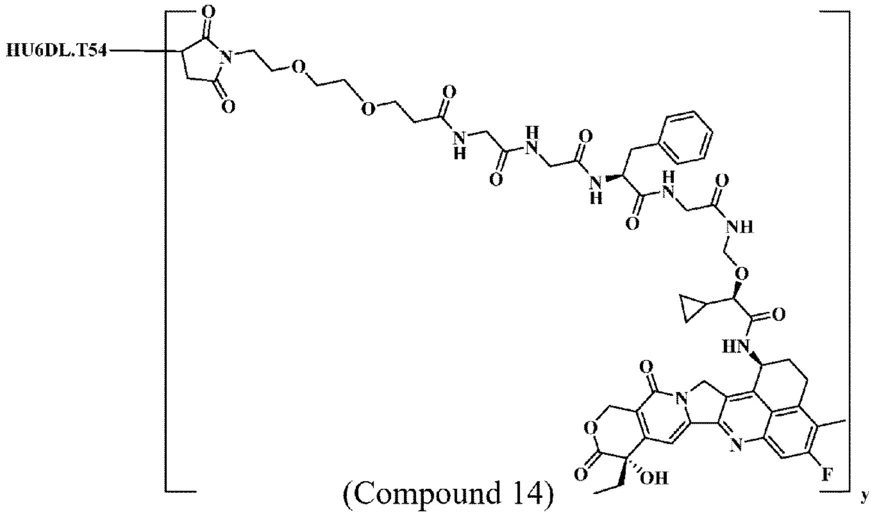

(Compound 14)

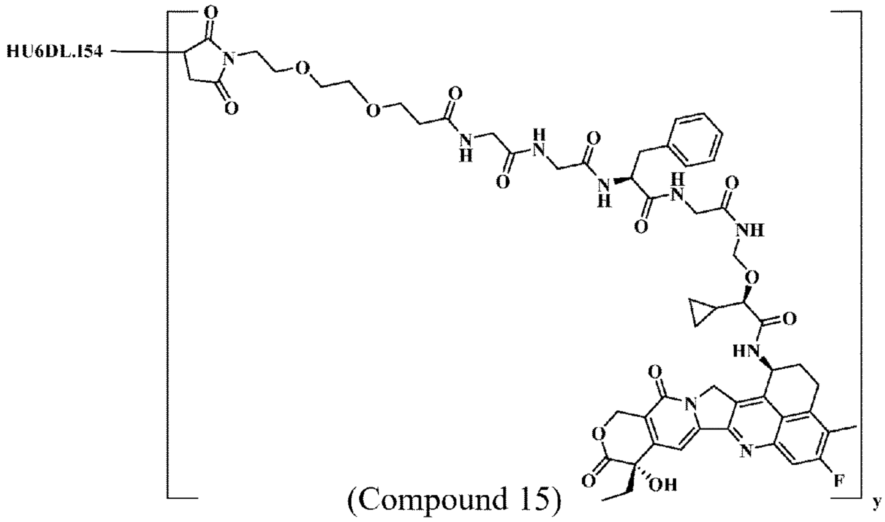

(Compound 15)

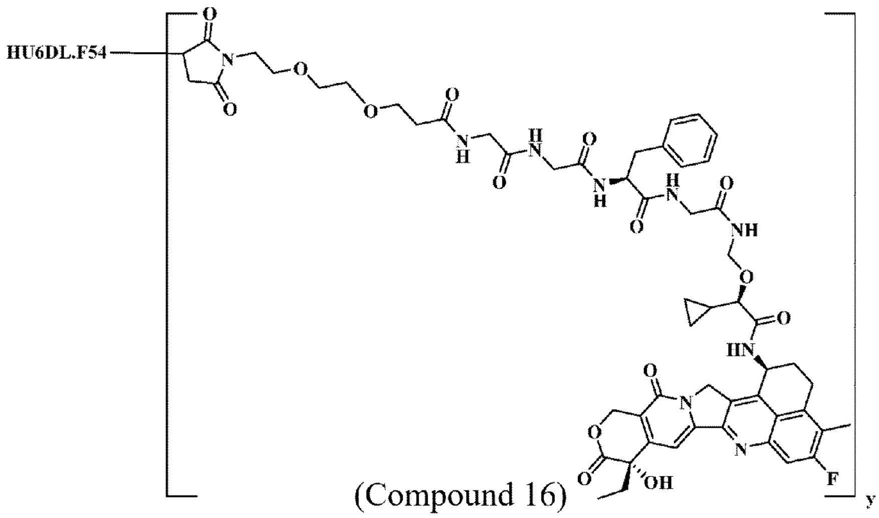

(Compound 16)

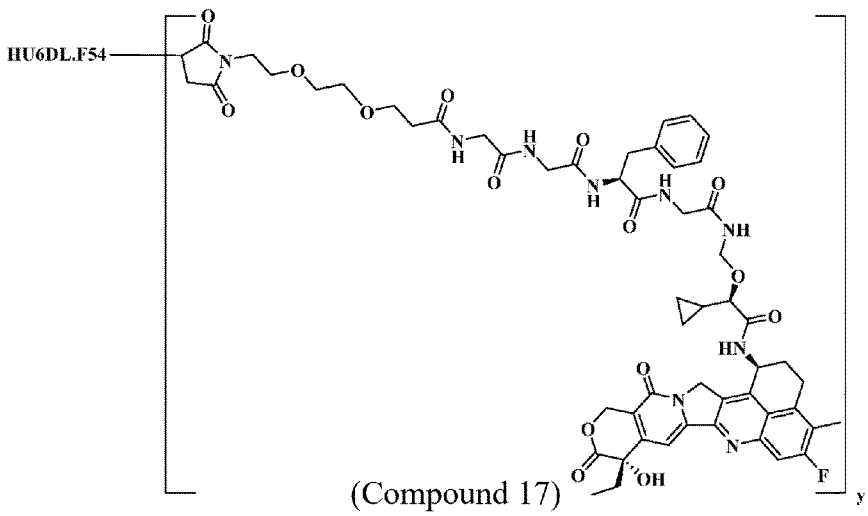

(Compound 17)

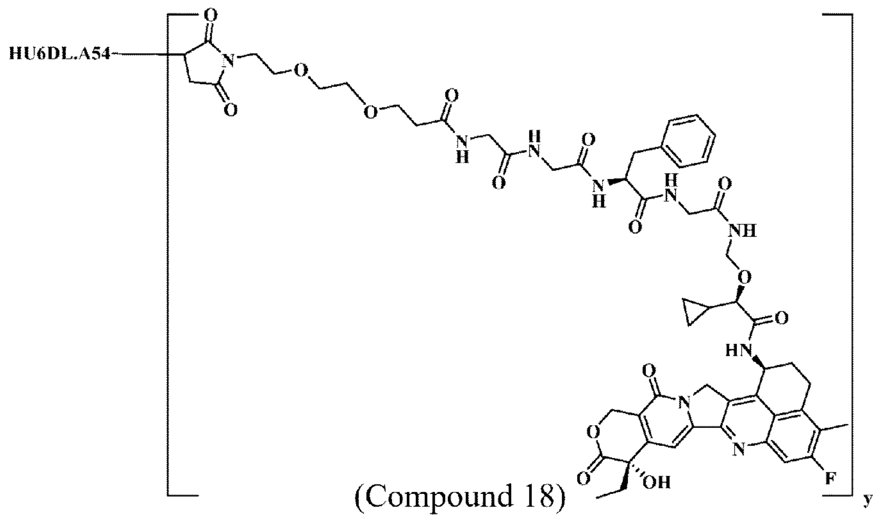

(Compound 18)

wherein y is as defined in claim 1.

**13.** A method for preparing an antibody-drug conjugate of general formula (I) or a pharmaceutically acceptable salt or solvent compound, comprising the following steps:

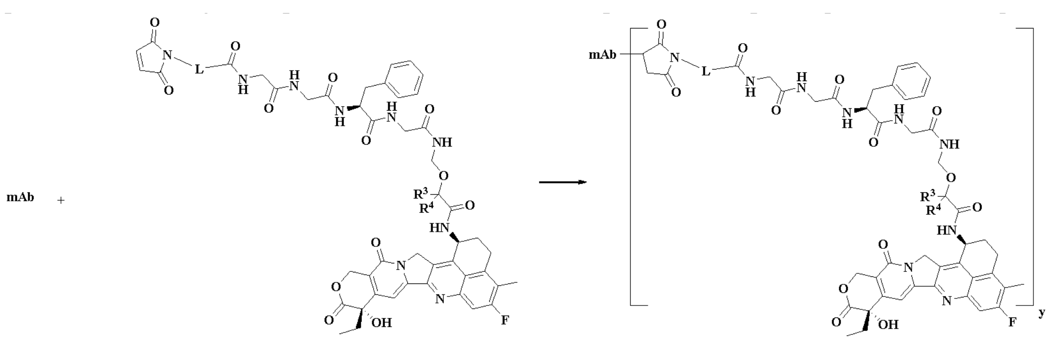

(F) (I)

reducing a mAb, and then conjugating the reduced mAb with a compound of general formula (F) to obtain the compound of general formula (I),
wherein L, $R^3$, R4, mAb and y are as defined in claim 1.

**14.** A pharmaceutical composition comprising the antibody-drug conjugate or the pharmaceutically acceptable salt or solvent compound thereof of any one of claims 1-12, and one or more pharmaceutically acceptable excipients, diluents or carriers.

**15.** Use of the antibody-drug conjugate or the pharmaceutically acceptable salt or solvent compound thereof according to any one of claims 1 to 12 or the pharmaceutical composition according to claim 14 in treatment of a TROP-2 related disease or condition.

**16.** The use according to claim 15, wherein, the human TROP-2 related disease is a cancer with high expression of TROP-2, and the cancer is selected from the group consisting of triple-negative breast cancer, small cell lung cancer, urothelial cancer, human brain astroblastoma, human pharyngeal cancer, adrenal gland tumors, AIDS-related cancers, alveolar soft tissue sarcoma, astrocytoma, bladder cancer, bone cancer, brain and spinal cord cancer, metastatic brain tumors, breast cancer, carotid body tumor, cervical cancer, chondrosarcoma, chordoma, chromophobe cell tumor of kidney, clear cell carcinoma, colon cancer, colorectal cancer, desmoplastic small round cell tumor, ependymoma, ewing tumour, extraskeletal myxoid chondrosarcoma, fibrous dysplasia of bone, osteofibrous dysplasia, gallbladder cancer or cholangiocarcinoma, stomach cancer, gestational trophoblastic disease, germ cell tumors, head and neck cancer, hepatocellular carcinoma, islet cell tumor, Kaposin's sarcoma, kidney cancer, leukemia, liposarcoma, malignant lipomatous tumors, liver cancer, lymphoma, lung cancer, medulloblastoma, melanoma, meningioma, multiple endocrine neoplasia, multiple myeloma, myelodysplastic syndrome, neuroblastoma, neuroendocrine tumors, ovarian cancer, pancreatic cancer, papillary thyroid cancer, parathyroidoma, pediatric cancer, peripheral nerve sheath tumors, pheochromocytoma, pituitary tumors, prostate cancer, posterior uveal melanoma, renal metastatic cancer, rhabdoid tumors, rhabdomyosarcoma, sarcoma, skin cancer, soft tissue sarcoma, squamous cell carcinoma, synovial sarcoma, testicular cancer, thymic cancer, thymoma, metastatic thyroid cancer and uterine cancer.

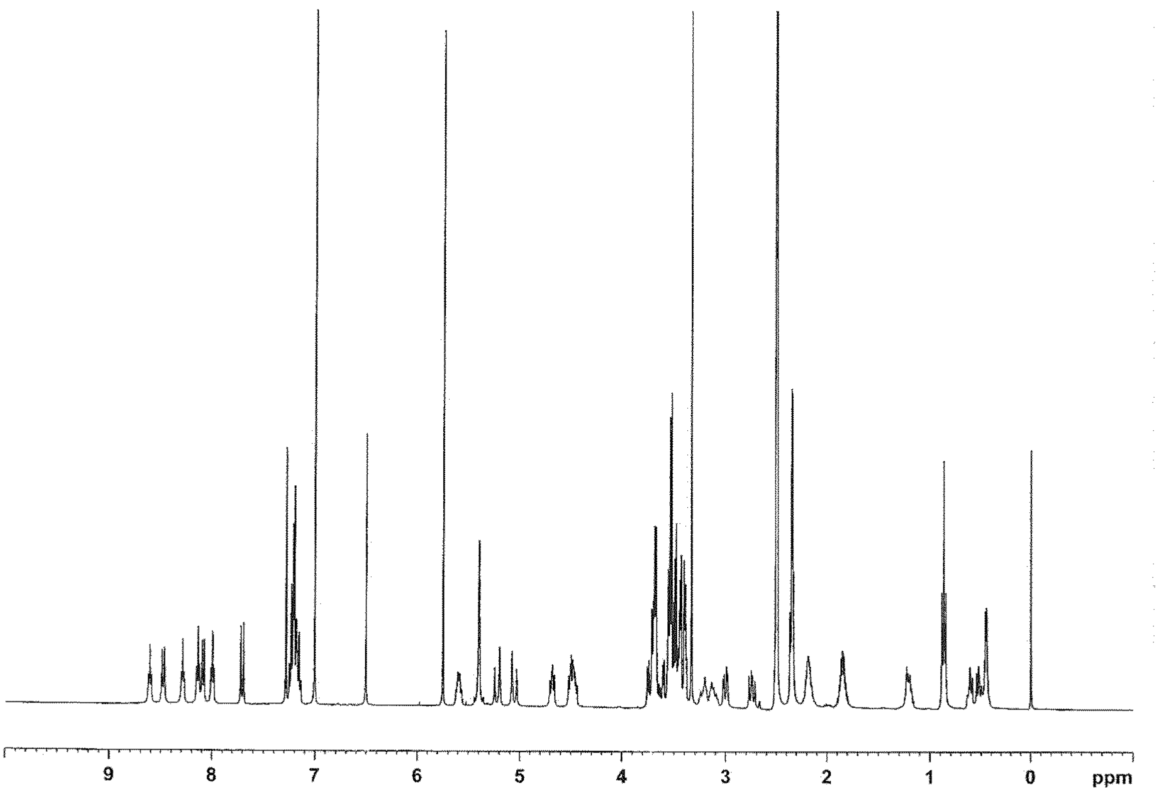
9
8
7
6
5
4
3
2
1
0
ppm

## INTERNATIONAL SEARCH REPORT

International application No. **PCT/CN2022/120481**

**A. CLASSIFICATION OF SUBJECT MATTER**

C07K 16/30(2006.01)i； A61K 47/68(2017.01)i； A61K 31/4745(2006.01)i； A61P 35/00(2006.01)i

According to International Patent Classification (IPC) or to both national classification and IPC

**B. FIELDS SEARCHED**

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

VEN, VCN, CJFD, Pubmed, ISI, 万方, WANFANG, CNKI, 读秀, DUXIU, STN, GenBank, 中国专利生物序列检索系统, Chinese Patent Biological Sequence Retrieval System: Trop2, Trop-2, ADC, antibody drug conjugate?, SEQ ID NOs: 3-17.

**C. DOCUMENTS CONSIDERED TO BE RELEVANT**

| Category* | Citation of document, with indication, where appropriate, of the relevant passages | Relevant to claim No. |
|---|---|---|
| A | WO 2020228604 A1 (JIANGSU HANSOH PHARMACEUTICAL GROUP CO., LTD. et al.) 19 November 2020 (2020-11-19)<br>entire document | 1-16 |
| A | CN 110903395 A (SICHUAN KELUN-BIOTECH BIOPHARMACEUTICA CO., LTD.) 24 March 2020 (2020-03-24)<br>entire document | 1-16 |
| A | CN 113039205 A (CARSGEN THERAPEUTICS CO., LTD.) 25 June 2021 (2021-06-25)<br>entire document | 1-16 |
| A | CN 107446050 A (BIO-THERA SOLUTIONS, LTD.) 08 December 2017 (2017-12-08)<br>entire document | 1-16 |
| A | CN 112168978 A (PEKING UNIVERSITY) 05 January 2021 (2021-01-05)<br>entire document | 1-16 |
| A | US 2013122020 A1 (RINAT NEUROSCIENCE CORP.) 16 May 2013 (2013-05-16)<br>entire document | 1-16 |
| A | ZAMAN, S. et al. "Targeting Trop-2 in solid tumors: future prospects" *OncoTargets and Therapy,* Vol. 12, 31 December 2019 (2019-12-31),<br>pp. 1781-1790 | 1-16 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **01 December 2022** | **15 December 2022** |

Name and mailing address of the ISA/CN

**China National Intellectual Property Administration (ISA/CN)**
**No. 6, Xitucheng Road, Jimenqiao, Haidian District, Beijing 100088, China**

Facsimile No. **(86-10)62019451**

Authorized officer

Telephone No.

Form PCT/ISA/210 (second sheet) (January 2015)

**INTERNATIONAL SEARCH REPORT**

International application No.
**PCT/CN2022/120481**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **15-16**
because they relate to subject matter not required to be searched by this Authority, namely:

[1] Claims 15-16 relate to using the antibody conjugate or a salt thereof to treat Trop-2 related diseases. That is, claims 15-16 relate to the subject matter as defined in PCT Rule 39.1 that does not warrant a search conducted by the international searching authority; and an international search was conducted on the basis of the technical solution of a use of the system in the preparation of a reagent for treating related diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/120481**

| Patent document cited in search report | Publication date (day/month/year) | Patent family member(s) | Publication date (day/month/year) |
|---|---|---|---|
| WO 2020228604 A1 | 19 November 2020 | TW 202108623 A | 01 March 2021 |
| | | CN 112243443 A | 19 January 2021 |
| CN 110903395 A | 24 March 2020 | None | |
| CN 113039205 A | 25 June 2021 | SG 11202104240 T A | 28 May 2021 |
| | | WO 2020083406 A1 | 30 April 2020 |
| | | CA 3117759 A1 | 30 April 2020 |
| | | EP 3875484 A1 | 08 September 2021 |
| | | KR 20210089179 A | 15 July 2021 |
| | | US 2021324087 A1 | 21 October 2021 |
| | | JP 2022505921 A | 14 January 2022 |
| CN 107446050 A | 08 December 2017 | CN 110526978 A | 03 December 2019 |
| | | CA 3038423 A1 | 14 February 2019 |
| | | AU 2018267660 A1 | 28 February 2019 |
| | | JP 2020503243 A | 30 January 2020 |
| | | CN 111087471 A | 01 May 2020 |
| | | EP 3464381 A1 | 10 April 2019 |
| | | US 2019048095 A1 | 14 February 2019 |
| | | CN 111018992 A | 17 April 2020 |
| | | CN 109078181 A | 25 December 2018 |
| | | WO 2019029715 A1 | 14 February 2019 |
| CN 112168978 A | 05 January 2021 | None | |
| US 2013122020 A1 | 16 May 2013 | RU 2014116406 A | 20 December 2015 |
| | | US 2014357844 A1 | 04 December 2014 |
| | | CA 2954166 A1 | 16 May 2013 |
| | | WO 2013068946 A2 | 16 May 2013 |
| | | KR 20140091040 A | 18 July 2014 |
| | | JP 2016104751 A | 09 June 2016 |
| | | US 2016257746 A1 | 08 September 2016 |
| | | CA 2854720 A1 | 16 May 2013 |
| | | BR 112014011331 A2 | 25 April 2017 |
| | | IL 232426 D0 | 30 June 2014 |
| | | TW 201333036 A | 16 August 2013 |
| | | SA 112330988 B1 | 22 July 2015 |
| | | CO 7010785 A2 | 31 July 2014 |
| | | US 2019002559 A1 | 03 January 2019 |
| | | AR 088693 A1 | 25 June 2014 |
| | | HK 1201851 A1 | 11 September 2015 |
| | | JP 2014534233 A | 18 December 2014 |
| | | MX 2014005728 A | 30 May 2014 |
| | | JP 2017141249 A | 17 August 2017 |
| | | EP 2776470 A2 | 17 September 2014 |
| | | ZA 201403492 B | 26 April 2017 |
| | | MX 317088 B | 13 January 2014 |
| | | SG 11201401699 W A | 26 September 2014 |
| | | AU 2012335205 A1 | 29 May 2014 |
| | | CN 104053672 A | 17 September 2014 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5840854 A
- US 6653104 B
- US 7420040 B
- CN 102827282 A
- CN 104114580 A
- WO 2020228604 A


### Non-patent literature cited in the description


- *J. Biol. Chem,* 1968, vol. 243, 3558
- **HOLLIGER ; HUDSON.** *Nat. Biotechnol.,* 2005, vol. 23, 1126-1136
- *Journal of Immunoglobulin,* 2001, ISBN 012441351